(19) **Europäisches Patentamt / European Patent Office / Office européen des brevets**

(11) **EP 2 914 740 B1**

(12) # EUROPEAN PATENT SPECIFICATION

(45) Date of publication and mention
of the grant of the patent:
**13.09.2017 Bulletin 2017/37**

(21) Application number: **13788920.0**

(22) Date of filing: **29.10.2013**

(51) Int Cl.:
**C12Q 1/68** (2006.01)

(86) International application number:
**PCT/EP2013/072603**

(87) International publication number:
**WO 2014/067943 (08.05.2014 Gazette 2014/19)**

(54) **METHOD OF DETECTING ACTIVE TUBERCULOSIS IN CHILDREN IN THE PRESENCE OF A CO-MORBIDITY**

VERFAHREN ZUR DETEKTION AKTIVER TUBERCULOSIS IN KINDERN IN DER ANWESENHEIT EINER KOMORBIDITÀT

PROCEDE DE DETECTION DE TUBERCULOSE ACTIVE DANS L'ENFANT EN PRESENCE D'UN COMORBITITE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.10.2012 GB 201219447**
**13.09.2013 GB 201316321**

(43) Date of publication of application:
**09.09.2015 Bulletin 2015/37**

(73) Proprietor: **Imperial Innovations Ltd**
**London SW7 2PG (GB)**

(72) Inventors:
• **ELEY, Brian**
**London SW7 2PG (GB)**
• **COIN, Lachlan**
**London SW7 2PG (GB)**
• **LEVIN, Michael**
**London SW7 2PG (GB)**
• **ANDERSON, Suzanne**
**London SW7 2PG (GB)**

(74) Representative: **Sterling IP**
**Orion House**
**Bessemer Road**
**Welwyn Garden City AL7 1HH (GB)**

(56) References cited:
**WO-A2-2007/011412     WO-A2-2009/158521**
**WO-A2-2011/066008**

• **ADITHYA CATTAMANCHI ET AL: "A transcriptional signature for active TB: Have we found the needle in the haystack?", PLOS MEDICINE, vol. 10, no. 10, 22 October 2013 (2013-10-22), page e1001538, XP055091754, DOI: 10.1371/journal.pmed.1001538**
• **LILLY M VERHAGEN ET AL: "A predictive signature gene set for discriminating active from latent tuberculosis in Warao Amerindian children", BMC GENOMICS, BIOMED CENTRAL LTD, LONDON, UK, vol. 14, no. 1, 1 February 2013 (2013-02-01), page 74, XP021140860, ISSN: 1471-2164, DOI: 10.1186/1471-2164-14-74**
• **MATTHEW P. R. BERRY ET AL: "An interferon-inducible neutrophil-driven blood transcriptional signature in human tuberculosis", NATURE, vol. 466, no. 7309, 19 August 2010 (2010-08-19), pages 973-977, XP055001768, ISSN: 0028-0836, DOI: 10.1038/nature09247**
• **EMIL LESHO ET AL: "Transcriptional responses of host peripheral blood cells to tuberculosis infection", TUBERCULOSIS, ELSEVIER, GB, vol. 91, no. 5, 7 July 2011 (2011-07-07), pages 390-399, XP028287344, ISSN: 1472-9792, DOI: 10.1016/J.TUBE.2011.07.002 [retrieved on 2011-07-14]**

**(Cont. next page)**

- J. MAERTZDORF ET AL: "Common patterns and disease-related signatures in tuberculosis and sarcoidosis", PROCEEDINGS OF THE NATIONAL ACADEMY OF SCIENCES, vol. 109, no. 20, 30 April 2012 (2012-04-30), pages 7853-7858, XP055033439, ISSN: 0027-8424, DOI: 10.1073/pnas.1121072109
- SARAH M FORTUNE ET AL: "Host transcription in active and latent tuberculosis", GENOME BIOLOGY, vol. 11, no. 9, 1 January 2010 (2010-01-01), page 135, XP055079346, ISSN: 1465-6906, DOI: 10.1186/gb-2010-11-9-135
- KAFOROU MYRSINI ET AL: "Host RNA signatures for diagnostics: An example from paediatric tuberculosis in Africa", JOURNAL OF INFECTION, ACADEMIC PRESS, LONDON, GB, vol. 69, 26 September 2014 (2014-09-26), XP029018562, ISSN: 0163-4453, DOI: 10.1016/J.JINF.2014.08.006
- SUZANNE T. ANDERSON ET AL: "Diagnosis of Childhood Tuberculosis and Host RNA Expression in Africa", NEW ENGLAND JOURNAL OF MEDICINE, THE - NEJM -, vol. 370, no. 18, 1 May 2014 (2014-05-01), pages 1712-1723, XP055316396, ISSN: 0028-4793, DOI: 10.1056/NEJMoa1303657

2

**Description**

The present disclosure relates to a method of distinguishing active TB in children in the presence of a complicating factor, for example, latent TB and/or co-morbidities, such as those that present similar symptoms to TB. The disclosure also relates to a gene signature employed in the said method and to a bespoke gene chip for use in the method. The disclosure further relates to use of known gene chips in the methods of the disclosure and kits comprising the elements required for performing the method. The disclosure also relates to use of the method to provide a composite expression score which can be used in the diagnosis of TB, particularly in a low resource setting.

**BACKGROUND**

An estimated 8.8 million new cases and 1.45 million deaths are caused by Tuberculosis, TB (short for tubercle bacillus) each year (World Health Organisation statistics 2011). TB is an infectious disease caused by various species of mycobacteria, typically *Mycobacterium tuberculosis.* Tuberculosis usually attacks the lungs but can also affect other parts of the body. It is spread through the air then people who have an active TB infection cough, sneeze or otherwise transmit their saliva. Most infections in humans result in an asymptomatic, latent infection and about one in ten latent infections eventually progress to active disease which, if left untreated, kills more than 50% of those infected. Immunosuppression and malnutrition are among the risk factors for developing active TB.

The classic symptoms are a chronic cough with blood-tinged sputum, fever, night sweats, and weight loss (the latter giving rise to the formerly prevalent colloquial term "consumption"). Infection of organs other than the lungs causes a wide range of symptoms. Treatment is difficult and requires long courses of multiple antibiotics. Antibiotic resistance is a growing problem with numbers of multi-drug-resistant tuberculosis cases on the rise. This is, in part, due to the length of treatment needed. Those infected with latent TB are typically asymptomatic and therefore either forget or decided not to take antibiotics. Those infected with active TB often cease treatment when the symptoms clear even though the infection remains.

While most adult TB is diagnosed by detection of acid-fast bacilli (AFB) on sputum microscopy, the majority of childhood TB cases are both smear and *Mycobacterium tuberculosis* (MTB) culture-negative and diagnosed solely on clinical grounds (Zar et al 2011; Perez-Velez et al 2012). This is problematic as symptoms and signs of childhood TB are non-specific and common to a range of other conditions (Marais et al 2012). Clinical scoring systems designed to aid diagnosis have not been validated against the 'gold standard' of culture-confirmed diagnosis and their diagnostic accuracy varies markedly (Hesseling et al 2002; Hatherill et al 2010; Pearce et al 2012).

Over-diagnosis and thus inappropriate treatment of children suspected of having TB is common (Cuevas et al 2012). Conversely, under-diagnosis and late initiation of TB therapy is an important contributor to poor outcome (Drobac et al 2012), and TB is often identified only when patients are critically ill or at post-mortem (Chintu et al 2002). Due to inadequate case detection, diagnosis is often made late or only on post-mortem (Chintu et al 2002; McNally et al 2007) and a large proportion of children suffering from TB are not appropriately treated. Children are more likely to develop severe forms of TB such as miliary TB, TB meningitis (TBM) and spinal TB (van Well et al 2009; Zar et al 2001; Cruz et al 2007; Graham et al 2009), resulting in high morbidity and mortality (van Well et al 2009; Swaminathan et al 2010).

Correct diagnosis is of utmost importance in the treatment of TB. The treatment regimens for active TB and latent TB are different and so it is important to diagnose the two conditions correctly in order to provide appropriate therapy.

While TB in adults is predominantly pulmonary and can be diagnosed by the detection of *M. tuberculosis* (MTB) in sputum by microscopy, culture, or molecular methods such as GeneXpert, the microbiological diagnosis of childhood TB is difficult due to the paucibacillary nature of the disease, frequent non-pulmonary disease and the additional complexity of obtaining sputum from young children (Perez-Velez et al 2002; McNally et al 2007; Marais et al 2006; Zar et al 2005). Current microbiological diagnostic approaches require hospital admission to obtain nasogastric lavage fluids, or the use of hypertonic saline-induced sputum aspirates, and are associated with considerable use of scarce hospital resources (Perez-Velez et al 2002; McNally et al 2007; Zar et al 2005). Even with optimal application of these invasive approaches, MTB detection in gastric aspirates or induced sputum is only achieved in less than 20% of clinically suspected TB cases (Perez-Velez et al 2002; Marais et al 2006; Nicol et al 2011). Furthermore, immunological approaches to diagnosis including tuberculin skin tests (TST) and interferon gamma release assays (IGRA) are insufficiently sensitive and cannot differentiate active disease from latent TB infection (LTBI) and so at best have an adjunctive role in the diagnosis of active TB in children (Machingaidze et al 2011; Mandalakas et al 2011; Madhi et al 2011).

Childhood TB may present either acutely or insidiously (McNally et al 2007; Marais et al 2006), with non-specific features such as failure to thrive, low grade fever, cough, weight loss, and lethargy and thus the difficulties in microbiological diagnosis are compounded by the clinical and radiological complexity of distinguishing TB from other common conditions such as pneumonia, malnutrition and malignancy.

As a consequence of these diagnostic challenges, a high proportion of children with suspected TB are treated solely on clinical suspicion, without any form of microbiological, radiological or immunological confirmation. Delayed or missed diagnosis as well as incorrect treatment is common and mortality rates for severe forms of childhood TB remain unacceptably high (McNally et al 2007; van Well et al 2009; Zar et al 2005; Shingadia et al 2012). The complexity of TB diagnosis is compounded in HIV-infected children in whom TB must be distinguished from a wide range of opportunistic infections. Furthermore T cell depletion in HIV-infected children increases the rate of skin test non-reactivity and further reduces the value of both TST and IGRA (Graham et al 2009; Eamranond et al 2001; Kampmann et al 2009).

RNA expression analysis by microarray has emerged as a powerful tool for understanding disease biology. Many diseases, including cancer and infectious diseases are associated with specific transcriptional profiles in blood or tissue.

In an influential study, Berry et al (2001) found a 393 transcript signature derived in an adult UK cohort that was able to distinguish TB from LTBI, and an 86 transcript signature able to distinguish TB from other inflammatory diseases. However, these signatures were derived from UK adult populations of HIV-uninfected individuals. Therefore these signatures are of limited application to children and in Africa, where HIV infection and LTBI are endemic.

Many previous TB diagnostic biomarker studies have focused on distinguishing adult patients with TB from healthy uninfected or LTBI (Maertzdorf et al 2011a 2011b, Jacobsen et al 2007) or have used other disease controls which are not representative of the real world clinical diseases from which TB needs to be distinguished in Africa (Maertzdorf et al 2012, Berry et al 2010). Furthermore, previous studies have excluded HIV co-infected patients who are in fact the group in which new diagnostics are most needed.

Thus there is a need to identify biomarkers that discriminate TB in children (paediatric TB, pTB) from other diseases prevalent in African populations, where the burden of the HIV/TB pandemic is greatest.

## SUMMARY OF THE INVENTION

The present disclosure provides a method for detecting active TB in children in a subject derived sample in the presence of a complicating factor, comprising the step of detecting the modulation of at least 95% of the genes in a signature selected from the group consisting of:

    a) a 42 gene signature shown in Table 3,
    b) a 51 gene signature shown in Table 4, and
    c) a combination of signatures a) and b).

Advantageously use of the appropriate signature in a method according to the present disclosure allows the robust and accurate identification of the presence of active TB or the differentiation of active TB from latent TB in children in the most relevant clinical setting, for example Africa. The detection is not prevented by co-morbidity in the patient, such as HIV or malaria. This is a huge step forward on the road to treating TB because it allows accurate diagnosis which, in turn, allows patients to be appropriately treated. Furthermore, the components for use in the method to detect active TB can be provided in a simple format for use in low resource and/or rural settings. Also herein described is a gene chip comprising one or more of the gene signatures selected from the group consisting of:

    a) 60 to 100% of a 42 gene signature shown in Table 3,
    b) 60 to 100% of a 51 gene signature shown in Table 4,
    c) a combination of signatures a) and b), and
    d) optionally one or more house-keeping genes.

In a further aspect the present disclosure includes use of a known or commercially available gene chip in the method of the present disclosure.

The genes signatures can be employed to robustly identify active TB or latent TB in children.

Advantageously the different expression patterns represented by the gene signatures employed in the method of the present disclosure correlate across geographic location and HIV infected status (i.e. positive or negative). That is to say, the method is applicable to different geographic locations regardless of the presence or absence of HIV.

In a further aspect the present disclosure provides the treatment of active TB or latent TB after diagnosis employing the method herein.

## BRIEF DESCRIPTION OF THE FIGURES

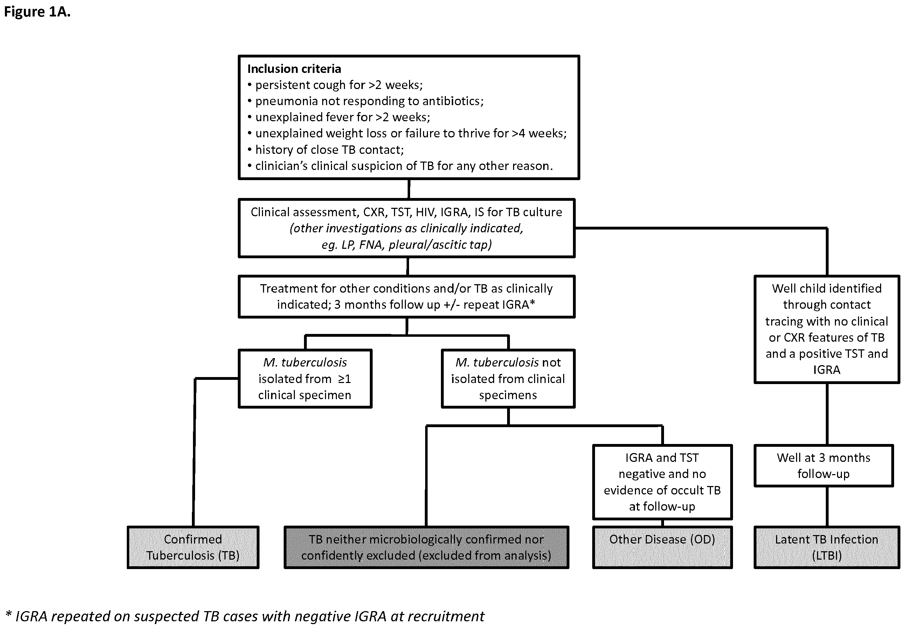

    **Figure 1A.** shows the diagnostic algorithm for suspected TB .

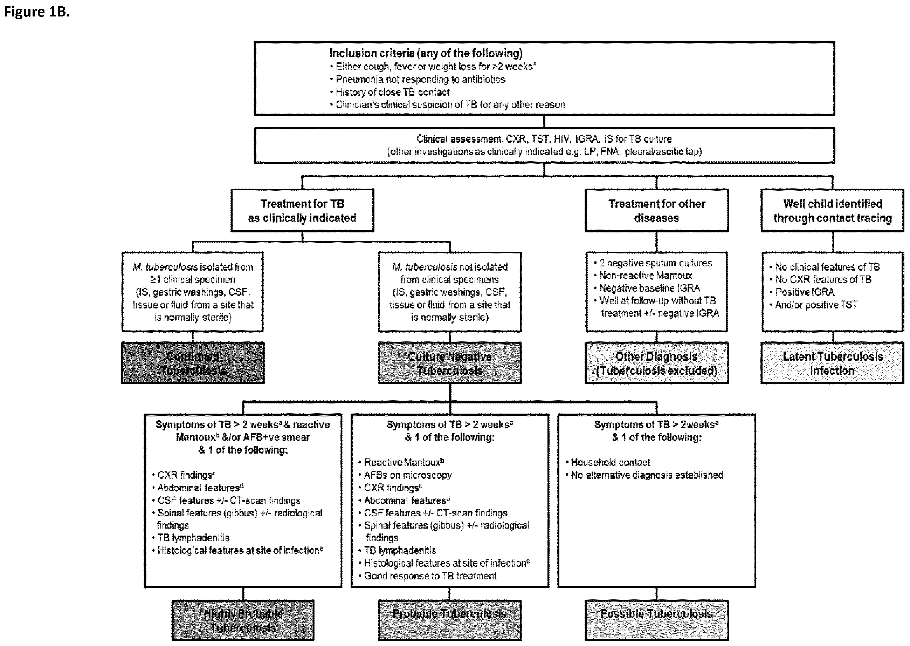

**Figure 1B.** shows an alternate diagnostic algorithm include culture negative patients. IS=induced sputum * failure to thrive for >4 weeks was included for the Kenyan cohort [a] IGRA repeated on suspected TB cases with negative IGRA at recruitment [b]≥10mm in HIV-ve and ≥5mum in HIV +ve [c] effusion, extensive consolidation, cavitation, lymphadenopathy, miliary, lobar pneumonia not responding to antibiotics [d] ascites, lymphadenopathy [e] e.g. caseating necrosis

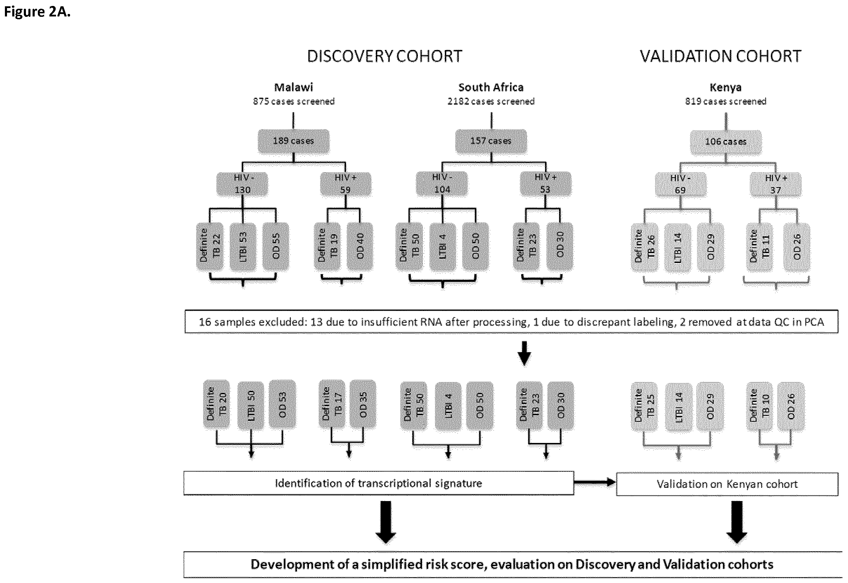

**Figure 2A.** shows the study overview showing patient numbers and analysis. HIV neg= HIV-uninfected, HIV pos= HIV-infected, TB= active tuberculosis, LTBI= latent TB infection, OD= other diseases (see Table 1B).

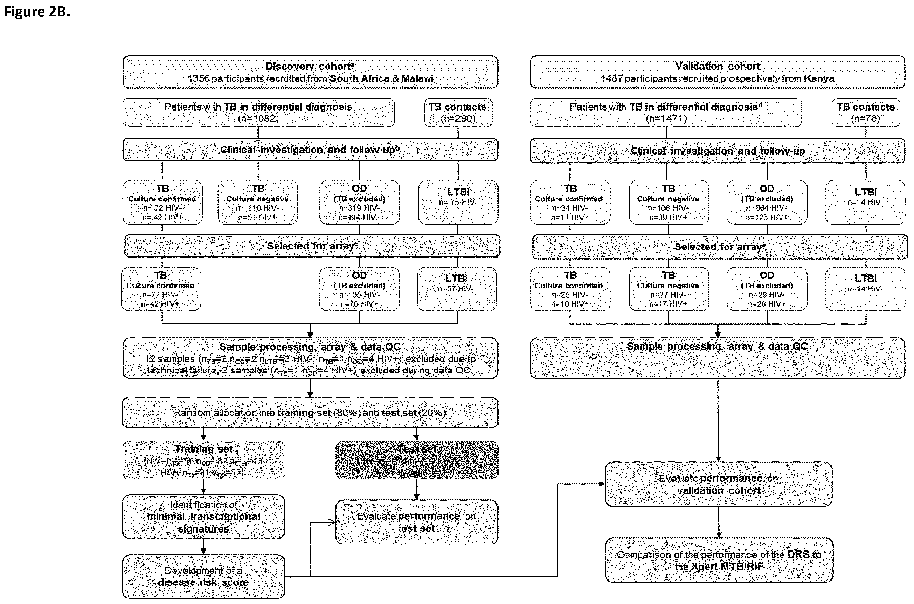

**Figure 2B.** shows an alternate study design with culture negative patients included in the validation cohort. [a] 16 excluded due to withdrawal of consent or inadequate samples collected. [b] samples excluded because of inconclusive diagnoses. [c] samples randomly selected. [d] this includes 60 TB contacts with features of TB on screening. [e] See examples for more details.

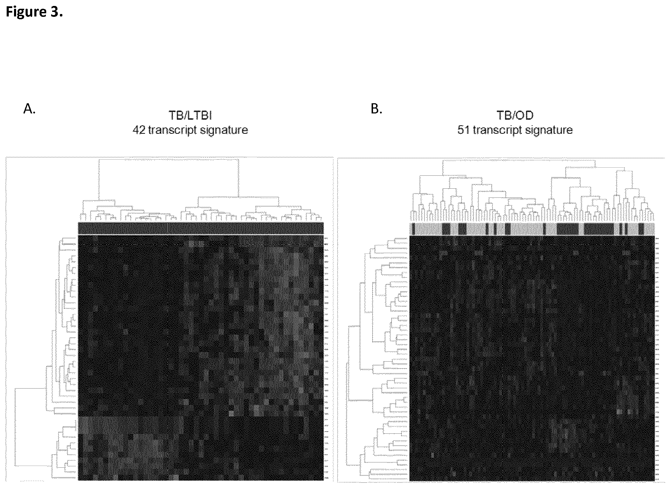

**Figure 3.** shows heatmap showing expression of transcripts identified by elastic net for TB vs. LTBI (A), and TB vs. OD (B). (nTB=87 nLTBI=43 / nTB=87 nOD=134) in Kenyan validation cohort. Rows are transcripts (transcripts shown in red are up-regulated, those in green are down-regulated) and columns are cases regardless of HIV status (TB cases - purple, LTBI - green, OD - light blue).

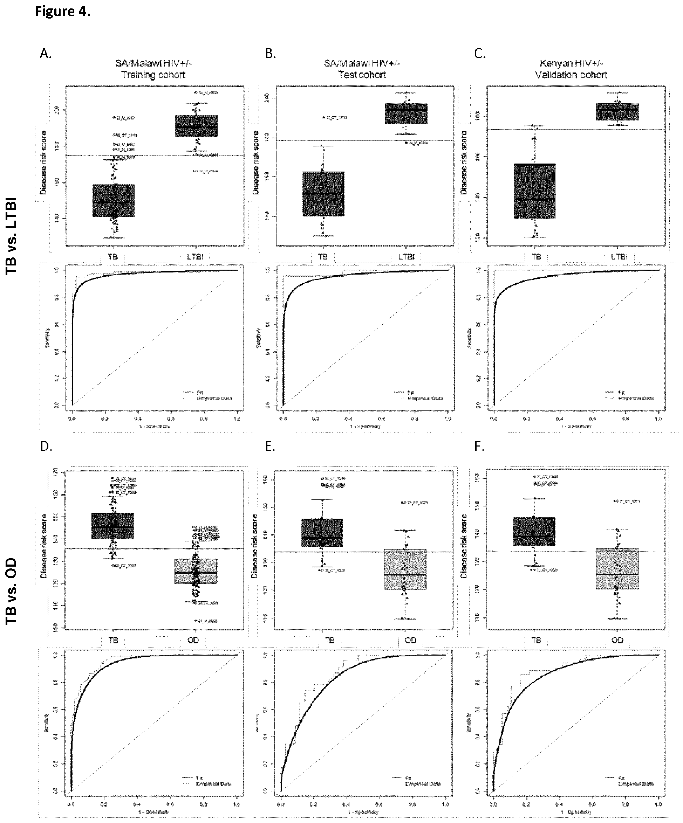

**Figure 4.** shows disease risk score and Receiver Operator Curves based on the TB/LTBI 42 transcript signature (A/B/C), the TB/OD 51 transcript signature (D/E/F) applied to the South African (SA)/Malawi HIV+/- training (A/D) and test (B/E) cohort (training: nTB=87 nLTBI=43 / nTB=87 nOD=134, test: nTB=23 nLTBI=11 / nTB=23 nOD=34) and independent Kenyan validation cohort (C/F) (nTB=35 nLTBI=14 / nTB=35 nOD=55). Sensitivity, specificity are reported in Table 2. HIV+ = HIV-infected, HIV-= HIV-uninfected. (TB cases - purple, LTBI - green, OD - light blue).

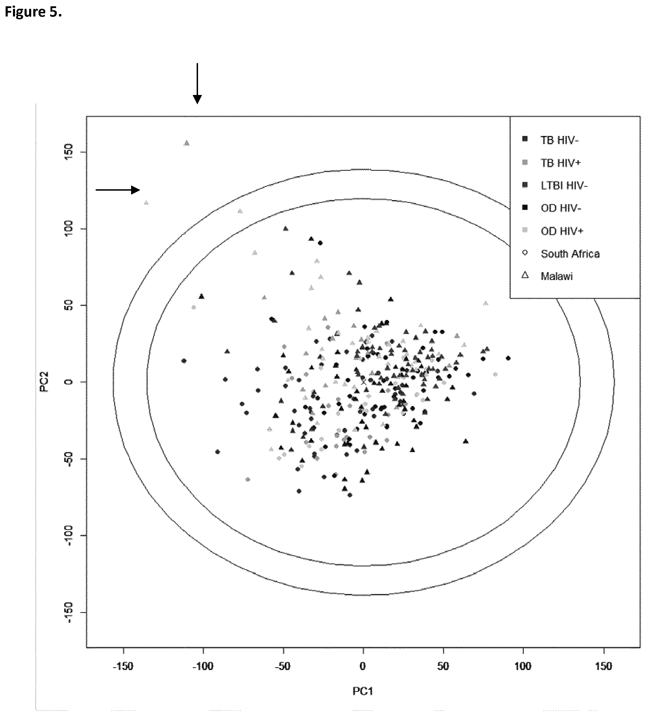

**Figure 5.** shows principal components analysis (PCA) of the microarrayed samples. PCA plot of PCA1 & PCA2 based on all genes on all of the samples after background adjustment and normalisation. The samples highlighted (categorised as TB/HIV+ and OD/HIV+ from Malawi) were removed from the analysis. Rings are levels of confidence (0.999 inner circle, 0.9999 outer circle).

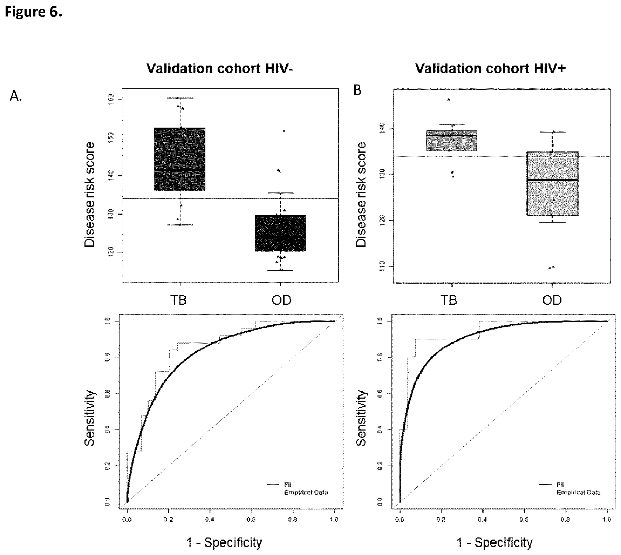

**Figure 6.** shows disease risk score and Receiver Operator Curves, based on the TB/OD 51 transcript signature applied to the independent Kenyan validation cohort separated by HIV status; A. HIV-(nTB=25 nOD=29) B. HIV+ (nTB=10 nOD=26). Sensitivity, specificity are reported in Table 2. HIV+ = = HIV-infected, HIV-= HIV-uninfected.

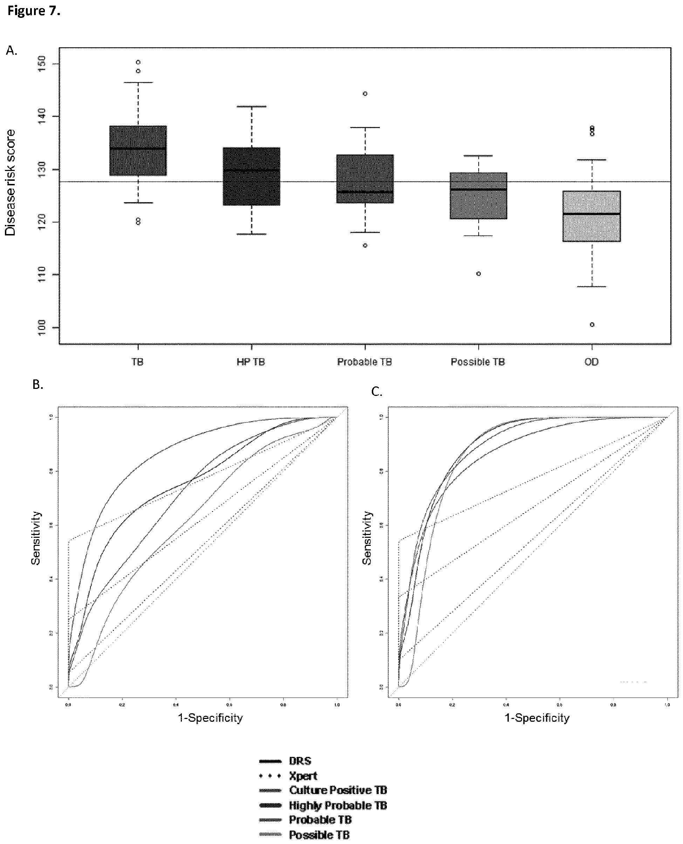

**Figure 7.** shows disease risk score based on the TB/OD 51-transcript signature applied to the independent Kenyan validation cohort (including culture negative patients) by clinical subgroup (A). Smoothed Receiver Operator Characteristic curves in the study population (B). Receiver Operator Characteristic curves using an adjusted sensitivity of 'actual' TB of 80% for the highly probable (HP), 50% for the probable and 40% for the possible TB cases (C). Sensitivity, specificity are reported in Table 2b. $n_{TB}$=35, $n_{OD}$=55, $n_{TB-HP}$=5, $n_{TB-PROBABLE}$= 19, $n_{TB-POSSIBLE}$=17.

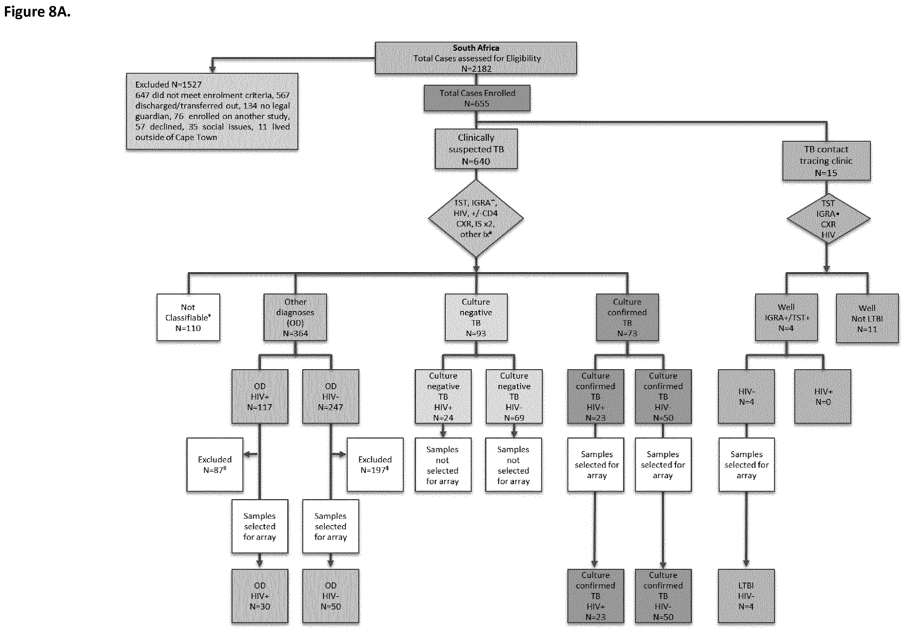

**Figure 8A.** shows recruitment at Red Cross War Memorial Children's Hospital, Cape Town, SA. ٭IGRA performed at baseline and 3 months in the OD category & at baseline and where possible 3 months in the TB cases category. ♯investigations done at attending clinician's discretion to diagnose OD's (urine, cerebrospinal fluid, blood cultures) as well as additional investigations performed to diagnose TB (ultrasound scans, CT-scans, histology and cytology). •1GRA performed at baseline and at 3 months. *cases excluded due to inconclusive/inadequate investigations at baseline. §cases excluded due to inconclusive diagnoses/patients lost to follow-up. Among HIV-uninfected and -infected definite TB cases, 76.0% and 56.5% of samples respectively were smear negative on microscopy.

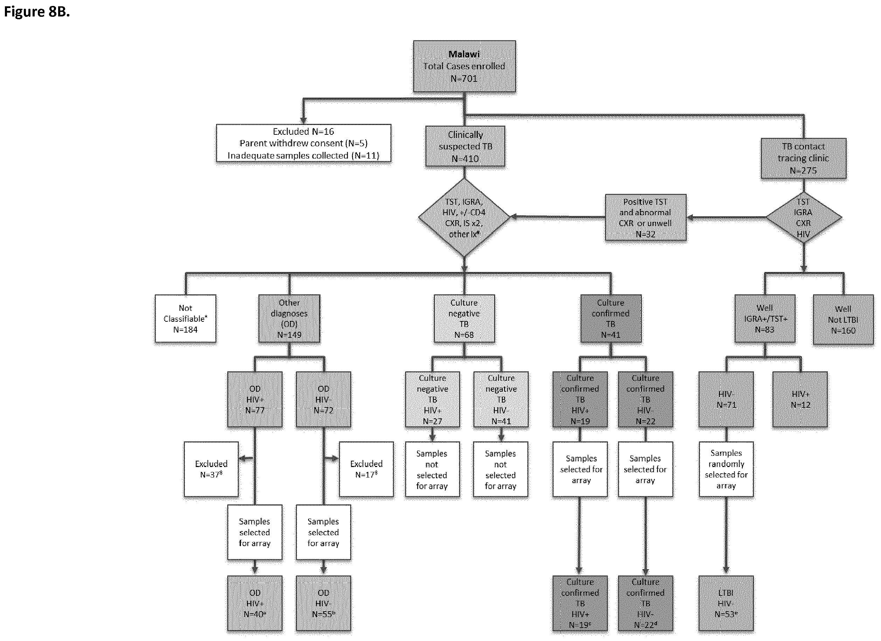

**Figure 8B.** shows recruitment at Queen Elizabeth Central Hospital, Blantyre, Malawi. ♯Investigations done at attending clinicians discretion to diagnose ODs (urine, CSF, blood cultures, histology, malaria thick film); additional investigations performed to diagnose TB (ultrasound scans, MRI-scans, TB blood culture, histology). *cases excluded due to inconclusive/inadequate investigations at baseline. § Samples excluded because of inconclusive diagnoses. [a,b,c,d,e] 4, 2, 1, 2, 3 samples respectively lost during sample processing. Among HIV-uninfected and -infected definite TB cases, 50% and 54% of samples respectively were smear negative on microscopy.

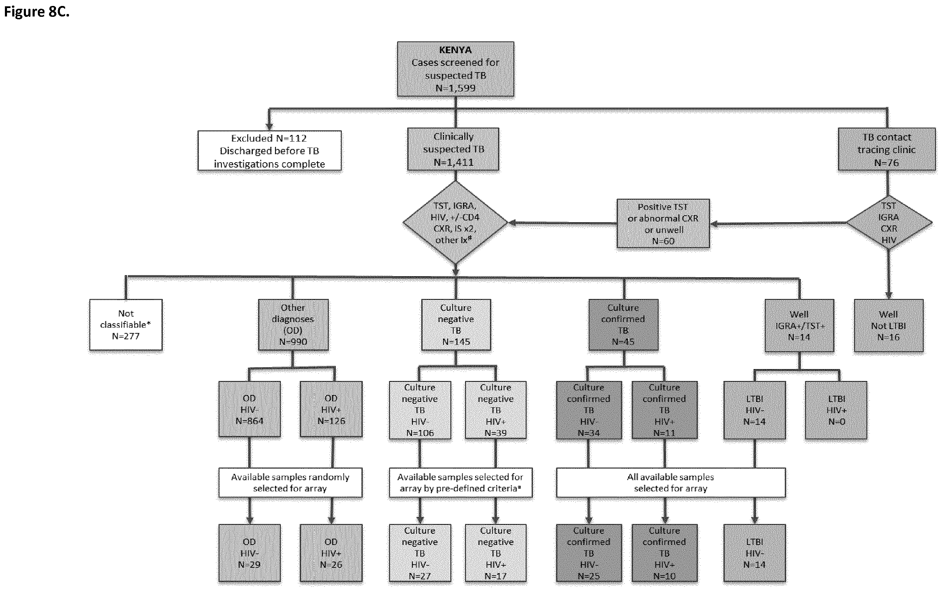

**Figure 8C.** shows Recruitment of validation cohort at Kilifi District Hospital & Coast Provincial General Hospital, Coast Province, Kenya. ♯Additional investigations done at the attending clinician's discretion to aid diagnosis of TB or ODs included: thick and thin films for malaria; blood cultures; urine cultures; CSF microscopy, culture, bacterial antigen tests and biochemistry; culture of pleural, peritoneal, joint and abscess fluid; bone marrow biopsy; radiological imaging including ultrasound and computed tomography scans; and tissue biopsy for histology and culture). *Cases that were not classifiable were those not treated for TB in whom TB could be neither diagnosed nor excluded with confidence due to death or loss to follow-up. [a] see examples.

For coloured versions of the figures refer to Anderson et al (NEJM - submitted 2013)

## DETAILED DESCRIPTION

Also herein described is the detection of the modulation of at least 60% of the genes in a signature such as 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100% providing the signature retains the ability to detect/discriminate the relevant clinical status without significant loss of specificity and/or sensitivity. The details of the gene signatures are given below.

In one embodiment the exact gene list in one or more of Tables 3 and 4 is employed. Also herein described is that the gene signature is the minimum set of genes required to optimally detect the infection or discriminate the disease.

Optimally is intended to mean the smallest set of genes needed to detect active TB in children without significant loss of specificity and/or sensitivity of the signature's ability to detect or discriminate.

Detect or detecting as employed herein is intended to refer to the process of identifying an active TB infection in a sample from a child, in particular through detecting modulation of the relevant genes in the signature.

Discriminate refers to the ability of the signature to differentiate between different disease status, for example latent and active TB. Detect and discriminate are interchangeable in the context of the gene signature. The method is able to detect an active TB infection in a sample.

Subject as employed herein is a human child suspected of TB infection from whom a sample is derived. The term patient may be used interchangeably although in one embodiment a patient has a morbidity.

Child as employed herein means a human of 18 years or less, for example 16 years or less, such as 1 month to 156 months. For example 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154 or 155 months.

Modulation of gene expression as employed herein means up-regulation or down-regulation of a gene or genes.

Up-regulated as employed herein is intended to refer to a gene transcript which is expressed at higher levels in a diseased or infected patient sample relative to, for example, a control sample free from a relevant disease or infection, or in a sample with latent disease or infection or a different stage of the disease or infection, as appropriate.

Down-regulated as employed herein is intended to refer to a gene transcript which is expressed at lower levels in a diseased or infected patient sample relative to, for example, a control sample free from a relevant disease or infection or in a sample with latent disease or infection or a different stage of the disease or infection.

The modulation is measured by measuring levels of gene expression by an appropriate technique. Gene expression as employed herein is the process by which information from a gene is used in the synthesis of a functional gene product. These products are often proteins, but in non-protein coding genes such as ribosomal RNA (rRNA), transfer RNA (tRNA) or small nuclear RNA (snRNA) genes, the product is a functional RNA. That is to say, RNA with a function.

A complicating factor as employed herein refers to at least one clinical status or at least one medical condition that would generally render it more difficult to identify the presence of active TB in the sample, for example a latent TB infection or a co-morbidity.

Co-morbidity as employed herein refers the presence of one or more disorders or diseases in addition to TB, for example malignancy such as cancer or co-infection. Co-morbidity may or may not be endemic in the general population.

In one embodiment the co-morbidity is a co-infection.

Co-infection as employed herein refers to bacterial infection, viral infection such as HIV, fungal infection and/or parasitic infection such as malaria. HIV infection as employed herein also extends to include AIDS.

In one embodiment other disease (OD) is a co-morbidity.

In one embodiment the 51 gene signature is able to detect active TB in the presence of a co-morbidity such as a co-infection. This is despite the increased inflammatory response of the patient to said other infection or co-infection.

In one embodiment co-morbidity is selected from malignancy, HIV, malaria, pneumonia, Lower Respiratory Tract Infection, Pneumocystis Jirovecii Pneumonia, pelvic inflammatory disease, Urinary Tract Infection, bacterial or viral meningitis, hepatobiliary disease, cryptococcal meningitis, non-TB pleural effusion, empyema, gastroenteritis, peritonitis, gastric ulcer and gastritis.

In one embodiment malignancy is a neoplasia, such as bronchial carcinoma, lymphoma, cervical carcinoma ovarian carcinoma, mesothelioma, gastric carcinoma, metastatic carcinoma, benign salivary tumour, dermatological tumour or Kaposi's sarcoma.

In one embodiment there is provided a method for detecting active TB in a subject derived sample in the presence of a complicating factor, comprising the step of detecting the modulation of at least 95% of the genes in a signature selected from the group consisting of:

a) a 42 gene signature shown in Table 3,
b) a 51 gene signature shown in Table 4,
c) a combination of signatures a) and b).

The 42 gene signature shown in Table 3 is useful in discriminating active TB infection from latent TB infection.

Active TB as employed herein refers to a child who is infected with TB which is not latent.

In one embodiment active TB is where the disease is progressing as opposed to where the disease is latent.

In one embodiment a child with active TB is capable of spreading the infection to others.

In one embodiment a child with active TB has one or more of the following: a skin test or blood test result indicating TB infection, an abnormal chest x-ray, a positive sputum smear or culture, active TB bacteria in his/her body, feels sick and may have symptoms such as coughing, fever, and weight loss. In one embodiment a child with active TB has one or more of the following symptoms: coughing, bloody sputum, fever and/or weight loss.

In one embodiment the active TB infection is pulmonary and/or extra-pulmonary.

Pulmonary as employed herein refers to an infection in the lungs.

Extra-pulmonary as employed herein refers to infection outside the lungs, for example, infection in the pleura, infection in the lymphatic system, infection in the central nervous system, infection in the genito-urinary tract, infection in the bones, infection in the brain and/or infection in the kidneys.

Symptoms of pulmonary TB include: a persistent cough that brings up thick phlegm, which may be bloody; breathlessness, which is usually mild to begin with and gradually gets worse; weight loss; lack of appetite; a high temperature of 38°C (100.4°F) or above; extreme tiredness; and a sense of feeling unwell.

Symptoms of lymph node TB include: persistent, painless swelling of the lymph nodes, which usually affects nodes in the neck, but swelling can occur in nodes throughout your body; over time, the swollen nodes can begin to release a discharge of fluid through the skin.

Symptoms of skeletal TB include: bone pain; curving of the affected bone or joint; loss of movement or feeling in the affected bone or joint and weakened bone that may fracture easily.

Symptoms of gastrointestinal TB include: abdominal pain; diarrhoea and anal bleeding.

Symptoms of genitourinary TB include: a burning sensation when urinating; blood in the urine; a frequent urge to pass urine during the night and groin pain.

Symptoms of central nervous system TB include: headaches; being sick; stiff neck; changes in your mental state, such as confusion; blurred vision and fits.

Latent TB as employed herein refers to a subject who is infected with TB but is asymptomatic. A sputum test will generally be negative and the infection cannot be spread to others.

In one embodiment a child with latent TB infection has one of more of the following: a skin test or blood test result indicating TB infection, a normal chest x-ray and a negative sputum test, TB bacteria in his/her body that are alive, but inactive, does not feel sick, cannot spread TB bacteria to others

In one embodiment a child with latent TB needs treatment to prevent TB disease becoming active.

In one embodiment the method of the present disclosure is able to differentiate TB from different conditions/diseases or infections which have similar clinical symptoms.

Similar symptoms as employed herein includes one or more symptoms from pulmonary TB, lymph node TB, skeletal TB, gastrointestinal TB, genitourinary TB and/or central nervous system TB.

In one embodiment the method according to the present disclosure is performed on a subject with acute infection.

In a further embodiment the sample is a subject sample from a febrile subject, that is to say with a temperature above the normal body temperature of 37.5°C.

Thus in one embodiment DNA or RNA from the subject sample is analysed.

In one embodiment the sample is solid or fluid, for example blood or serum or a processed form of any one of the same.

A fluid sample as employed herein refers to liquids originating from inside the bodies of living people. They include fluids that are excreted or secreted from the body as well as body water that normally is not. Includes amniotic fluid, aqueous humour and vitreous humour, bile, blood serum, breast milk, cerebrospinal fluid, cerumen (earwax), chyle, endolymph and perilymph, gastric juice, mucus (including nasal drainage and phlegm), sputum, peritoneal fluid, pleural fluid, saliva, sebum (skin oil), semen, sweat, tears, vaginal secretion, vomit, urine. Particularly blood and serum.

Blood as employed herein refers to whole blood, that is serum, blood cells and clotting factors, typically peripheral whole blood.

Serum as employed herein refers to the component of whole blood that is not blood cells or clotting factors. It is plasma with fibrinogens removed.

In one embodiment the subject derived sample is a blood sample.

In one or more embodiments the analysis is *ex vivo.*

*Ex vivo* as employed herein means that which takes place outside the body.

In one embodiment the 42 gene signature comprises of at least up-regulated genes APOL6, CLIP1, GBP6, RAP1A, CARD16, GBP5, DEFA1, ACTA2, DEFA1B, DEFA3 and LOC400759.

In one embodiment the 42 gene signature comprises of at least down-regulated genes NDRG2, UBA52, PHF17, SNHG7, C20ORF201, LOC389816, NOG, HS.538100, C8ORF55, C11ORF2, ALKBH7, KLHL28, GNG3, E4F1, LCMT1, TGIF1, PAQR7, C21ORF57, PASK, IMPDH2, PASK, LGTN, CRIP2, DGCR6, SIVA, LRRN3, DNAJC30, NME3, U2AF1L4, MFGE8 and FBLN5.

In one embodiment the 42 gene signature comprises of at least up-regulated genes APOL6, CLIP1, GBP6, RAP1A, CARD16, GBP5, DEFA1, ACTA2, DEFA1B, DEFA3 and LOC400759 and optionally down-regulated genes NDRG2, UBA52, PHF17, SNHG7, C20ORF201, LOC389816, NOG, HS.538100, C8ORF55, C11ORF2, ALKBH7, KLHL28, GNG3, E4F1, LCMT1, TGIF1, PAQR7, C21ORF57, PASK, IMPDH2, PASK, LGTN, CRIP2, DGCR6, SIVA, LRRN3, DNAJC30, NME3, U2AF1L4, MFGE8 and FBLN5.

In one embodiment the 51 gene signature comprises of at least up-regulated genes CYB561, GBP6, HS.106234, CCDC52, GBP3, LOC642678, ALDH1A1, CD226, SNORD8, LOC389386, TPST1, PDCD1LG2, SMARCD3, C1QB, CD79A, FER1L3, TNFRSF17, LOC389386, CYB561,KLHDC8B, SIGLEC14, OSBPL10, HLA-DRB6, HS.171481, CAST, F2RL1, HLA-DRB1, GBP5, ALAS2, KIFC3, HLA-DRB5, DEFA1 and NCF1B.

In one embodiment the 51 gene signature comprises of at least down-regulated genes VAMP5, C20ORF103, ZBED2, SEMA6B, CDKN1C, JUP, C3HC4, FRMD3, SCGB3A1, GRAMD1B, CEACAM1, LOC653778, KCNJ15, LOC649210, KREMEN1, HPSE, MIR1974 and LOC647460.

In one embodiment the 51 gene signature comprises of at least up-regulated genes CYB561, GBP6,, S.106234, CCDC52, GBP3, LOC642678, ALDH1A1, CD226, SNORD8, LOC389386, TPST1, PDCD1LG2, SMARCD3, C1QB, CD79A, FER1L3, TNFRSF17, LOC389386, CYB561,KLHDC8B, SIGLEC14, OSBPL10, HLA-DRB6, HS.171481, CAST, F2RL1, HLA-DRB1, GBP5, ALAS2, KIFC3, HLA-DRB5, DEFA1 and NCF1B and optionally down-regulated genes VAMP5, C20ORF103, ZBED2, SEMA6B, CDKN1C, JUP, C3HC4, FRMD3, SCGB3A1, GRAMD1B, CEACAM1, LOC653778, KCNJ15, LOC649210, KREMEN1, HPSE, MIR1974 and LOC647460.

In one embodiment the 42 and 51 gene signatures are tested in parallel.

In one embodiment each of the genes in the 42 and 51 gene signatures is significantly differentially expressed in the sample with active TB compared to a comparator group.

Significantly differentially expressed as employed herein means the sample with active TB shows a log2 fold change >0.5.

In the 42 gene signature the comparator group is LTBI.

In the 51 gene signature the comparator group is a child with "other disease" (OD), that is a disease that is not active TB but has similar symptoms.

"Presented in the form of" as employed herein refers to the laying down of genes from one or more of the signatures in the form of probes on a microarray.

Accurately and robustly as employed herein refers to the fact that the method can be employed in a practical setting, such as Africa, and that the results of performing the method properly give a high level of confidence that a true result is obtained.

High confidence is provided by the method when it provides few results that are false positives (i.e. the result suggests that the subject has active TB when they do not) and also has few false negatives (i.e. the result suggest that the subject does not have active TB when they do).

High confidence would include 90% or greater confidence, such as 91, 92, 93, 94, 95, 96, 97, 98, 99 or 100% confidence when an appropriate statistical test is employed.

In one embodiment the method provides a sensitivity of 80% or greater such as 90% or greater in particular 95% or greater, for example where the sensitivity is calculated as below:

$$\text{sensitivity} = \frac{\text{number of true positives}}{\text{number of true positives} + \text{number of false negatives}}$$

$$= \text{probability of a positive test given that the patient is ill}$$

8

In one embodiment the method provides a high level of specificity, for example 80% or greater such as 90% or greater in particular 95% or greater, for example where specificity is calculated as shown below:

$$\text{specificity} = \frac{\text{number of true negatives}}{\text{number of true negatives} + \text{number of false positives}}$$

$$= \text{probability of a negative test given that the patient is well}$$

In one embodiment the sensitivity of method of the 42 gene signature is 85 to 100%, such as 86, 87, 88, 89, 90, 91, 92, 93, 94, 95, 96, 97, 98 or 99%.

In one embodiment the specificity of the method of the 42 gene signature is 72 to 100%, such as 73, 74, 75, 76, 77, 78, 79, 80, 81, 82, 83, 84, 85, 86, 87, 88, 89, 90, 91, 92, 93, 94, 95, 96, 97, 98 or 99%.

In one embodiment the sensitivity of the method of the 51 gene signature is 55 to 100%, such as 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 or 99%.

In one embodiment the specificity of the method of the 51 gene signature is 55 to 100%, such as 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 or 99%.

There are a number of ways in which gene expression can be measured including microarrays, tiling arrays, DNA or RNA arrays for example on gene chips, RNA-seq and serial analysis of gene expression.

Any suitable method of measuring gene modulation may be employed in the method of the present disclosure.

In one embodiment the gene expression data is generated from a microarray, such as a gene chip.

Microarray as employed herein includes RNA or DNA arrays, such as RNA arrays.

A gene chip is essentially a microarray that is to say an array of discrete regions, typically nucleic acids, which are separate from one another and are, for example arrayed at a density of between, about 100/cm$^2$ to 1000/cm$^2$, but can be arrayed at greater densities such as 10000/cm$^2$.

The principle of a microarray experiment, is that mRNA from a given cell line or tissue is used to generate a labelled sample typically labelled cDNA or cRNA, termed the 'target', which is hybridised in parallel to a large number of, nucleic acid sequences, typically DNA or RNA sequences, immobilised on a solid surface in an ordered array. Tens of thousands of transcript species can be detected and quantified simultaneously. Although many different microarray systems have been developed the most commonly used systems today can be divided into two groups.

Using this technique, arrays consisting of more than 30,000 cDNAs can be fitted onto the surface of a conventional microscope slide. For oligonucleotide arrays, short 20-25mers are synthesised in situ, either by photolithography onto silicon wafers (high-density-oligonucleotide arrays from Affymetrix) or by ink-jet technology (developed by Rosetta Inpharmatics and licensed to Agilent Technologies). Alternatively, pre-synthesised oligonucleotides can be printed onto glass slides. Methods based on synthetic oligonucleotides offer the advantage that because sequence information alone is sufficient to generate the DNA to be arrayed, no time-consuming handling of cDNA resources is required. Also, probes can be designed to represent the most unique part of a given transcript, making the detection of closely related genes or splice variants possible. Although short oligonucleotides may result in less specific hybridisation and reduced sensitivity, the arraying of pre-synthesised longer oligonucleotides (50-100mers) has recently been developed to counteract these disadvantages.

In one example described herein the gene chip is an off the shelf, commercially available chip, for example HumanHT-12 v4 Expression BeadChip Kit, available from Illumina, NimbleGen microarrays from Roche, Agilent, Eppendorf and Genechips from Affymetrix such as HU-UI 33.Plus 2.0 gene chips.

In an alternate example described herein the gene chip employed in the present invention is a bespoke gene chip, that is to say the chip contains only the target genes which are relevant to the desired profile. Custom made chips can be purchased from companies such as Roche, Affymetrix and the like. In yet a further embodiment the bespoke gene chip comprises a minimal disease specific transcript set.

In one embodiment the chip consists of 100% of the 42 genes listed in Table 3.

In one embodiment the chip consists of 100% of the 51 genes listed in Table 4.

In one embodiment the chip consists of 100% of the 42 genes listed in Table 3 in combination with 100% of the 51 genes listed in Table 4.

In one or more embodiments above the chip may further include 1 or more, such as 1 to 10, house-keeping genes.

In one embodiment the gene expression data is generated in solution using appropriate probes for the relevant genes.

Probe as employed herein is intended to refer to a hybridisation probe which is a fragment of DNA or RNA of variable length (usually 100-1000 bases long) which is used in DNA or RNA samples to detect the presence of nucleotide sequences (the DNA target) that are complementary to the sequence in the probe. The probe thereby hybridises to single-stranded nucleic acid (DNA or RNA) whose base sequence allows probe-target base pairing due to complementarity between the probe and target.

In one embodiment the method according to the present disclosure and for example chips employed therein may comprise one or more house-keeping genes. House-keeping genes as employed herein is intended to refer to genes that are not directly relevant to the profile for identifying the disease or infection but are useful for statistical purposes and/or quality control purposes, for example they may assist with normalising the data, in particular a house-keeping gene is a constitutive gene i.e. one that is transcribed at a relatively constant level. The housekeeping gene's products are typically needed for maintenance of the cell. Examples include actin, GAPDH and ubiquitin. Minimal disease specific transcript set as employed herein means the minimum number of genes need to robustly identify the target disease state.

Minimal discriminatory gene set is interchangeable with minimal disease specific transcript set. Normalising as employed herein is intended to refer to statistically accounting for background noise by comparison of data to control data, such as the level of fluorescence of house-keeping genes, for example fluorescent scanned data may be normalised using RMA to allow comparisons between individual chips. Irizarry et al 2003 describes this method.

Scaling as employed herein refers to boosting the contribution of specific genes which are expressed at low levels or have a high fold change but still relatively low fluorescence such that their contribution to the diagnostic signature is increased.

Fold change is often used in analysis of gene expression data in microarray and RNA-Seq experiments, for measuring change in the expression level of a gene and is calculated simply as the ratio of the final value to the initial value i.e. if the initial value is A and final value is B, the fold change is B/A. Tusher et al 2001.

In programs such as Arrayminer, fold change of gene expression can be calculated. The statistical value attached to the fold change is calculated and is the more significant in genes where the level of expression is less variable between subjects in different groups and, for example where the difference between groups is larger.

The step of obtaining a suitable sample from the subject is a routine technique, which involves taking a blood sample. This process presents little risk to donors and does not need to be performed by a doctor but can be performed by appropriately trained support staff. In one embodiment the sample derived from the subject is approximately 2.5 ml of blood, however smaller volumes can be used for example 0.5-1ml.

Blood or other tissue fluids are immediately placed in an RNA stabilizing buffer such as included in the Pax gene tubes, or Tempus tubes.

If storage is required then it should usually be frozen within 3 hours of collections at -80°C.

In one embodiment the gene expression data is generated from RNA levels in the sample.

For microarray analysis the blood may be processed using a suitable product, such as PAX gene blood RNA extraction kits (Qiagen).

Total RNA may also be purified using the Tripure method - Tripure extraction (Roche Cat. No. 1 667 165). The manufacturer's protocols may be followed. This purification may then be followed by the use of an RNeasy Mini kit - clean-up protocol with DNAse treatment (Qiagen Cat. No. 74106). Quantification of RNA may be completed using optical density at 260nm and Quant-IT RiboGreen RNA assay kit (Invitrogen - Molecular probes RI 1490). The Quality of the 28s and 18s ribosomal RNA peaks can be assessed by use of the Agilent bioanalyser.

In another embodiment the method further comprises the step of amplifying the RNA. Amplification may be performed using a suitable kit, for example TotalPrep RNA Amplification kits (Applied Biosystems).

In one embodiment an amplification method may be used in conjunction with the labelling of the RNA for microarray analysis. The Nugen 3' ovation biotin kit (Cat: 2300-12, 2300-60).

The RNA derived from the subject sample is then hybridised to the relevant probes, for example which may be located on a chip. After hybridisation and washing, where appropriate, analysis with an appropriate instrument is performed.

In performing an analysis to ascertain whether a subject presents a gene signature indicative of disease or infection according to the present disclosure, the following steps are performed: obtain mRNA from the sample and prepare nucleic acids targets, hybridise to the array under appropriate conditions, typically as suggested by the manufactures of the microarray (suitably stringent hybridisation conditions such as 3X SSC, 0.1% SDS, at 50 <0>C) to bind corresponding probes on the array, and wash if necessary to remove unbound nucleic acid targets and analyse the results.

In one embodiment the readout from the analysis is fluorescence.

In one embodiment the readout from the analysis is colorimetric.

In one embodiment physical detection methods, such as changes in electrical impedance, nanowire technology or microfluidics may be used.

In one embodiment there is provided a method which further comprises the step of quantifying RNA from the subject sample.

If a quality control step is desired, software such as Genome Studio software may be employed.

Numeric value as employed herein is intended to refer to a number obtained for each relevant gene, from the analysis or readout of the gene expression, for example the fluorescence or colorimetric analysis. The numeric value obtained from the initial analysis may be manipulated, corrected and if the result of the processing is a still a number then it will be continue to be a numeric value.

By converting is meant processing of a negative numeric value to make it into a positive value or processing of a positive numeric value to make it into a negative value by simple conversion of a positive sign to a negative or vice versa.

Analysis of the subject-derived sample for the genes analysed will give a range of numeric values some of which are positive (preceded by + and in mathematical terms considered greater than zero) and some of which are negative (preceded by - and in strict mathematical terms are considered to less than zero). The positive and negative in the context of gene expression analysis is a convenient mechanism for representing genes which are up-regulated and genes which are down regulated.

In the method of the present disclosure either all the numeric values of genes which are down-regulated and represented by a negative number are converted to the corresponding positive number (i.e. by simply changing the sign) for example -1 would be converted to 1 or all the positive numeric values for the up-regulated genes are converted to the corresponding negative number.

The present inventors have established that this step of rendering the numeric values for the gene expressions positive or alternatively all negative allows the summating of the values to obtain a single value that is indicative of the presence of disease or infection or the absence of the same.

This is a huge simplification of the processing of gene expression data and represents a practical step forward thereby rendering the method suitable for routine use in the clinic.

By discriminatory power is meant the ability to distinguish between a TB infected and a non-infected sample (subject) or between active TB infection and other infections (such as HIV) in particular those with similar symptoms or between a latent infection and an active infection.

The discriminatory power of the method according to the present disclosure may, for example, be increased by attaching greater weighting to genes which are more significant in the signature, even if they are expressed at low or lower absolute levels.

As employed herein, raw numeric value is intended to, for example refer to unprocessed fluorescent values from the gene chip, either absolute fluorescence or relative to a house keeping gene or genes.

Summating as employed herein is intended to refer to act or process of adding numerical values.

Composite expression score as employed herein means the sum (aggregate number) of all the individual numerical values generated for the relevant genes by the analysis, for example the sum of the fluorescence data for all the relevant up and down regulated genes. The score may or may not be normalised and/or scaled and/or weighted.

In one embodiment the composite expression score is normalised.

In one embodiment the composite expression score is scaled.

In one embodiment the composite expression score is weighted.

Weighted or statistically weighted as employed herein is intended to refer to the relevant value being adjusted to more appropriately reflect its contribution to the signature.

In one embodiment the method employs a simplified risk score as employed in the examples herein.

Control as employed herein is intended to refer to a positive (control) sample and/or a negative (control) sample which, for example is used to compare the subject sample to, and/or a numerical value or numerical range which has been defined to allow the subject sample to be designated as positive or negative for disease/infection by reference thereto.

Positive control sample as employed herein is a sample known to be positive for the pathogen or disease in relation to which the analysis is being performed, such as active TB.

Negative control sample as employed herein is intended to refer to a sample known to be negative for the pathogen or disease in relation to which the analysis is being performed.

In one embodiment the control is a sample, for example a positive control sample or a negative control sample, such as a negative control sample.

In one embodiment the control is a numerical value, such as a numerical range, for example a statistically determined range obtained from an adequate sample size defining the cut-offs for accurate distinction of disease cases from controls.

**Conversion of multi-gene transcript disease signatures into a single number disease score**

Once the RNA expression signature of the disease has been identified by variable selection, the transcripts are separated based on their up- or down-regulation relative to the comparator group. The two groups of transcripts are selected and collated separately.

## Summation of up-regulated and down-regulated RNA transcripts

To identify the single disease risk score for any individual patient, the raw intensities, for example fluorescent intensities (either absolute or relative to housekeeping standards) of all the up-regulated RNA transcripts associated with the disease are summated. Similarly summation of all down-regulated transcripts for each individual is achieved by combining the raw values (for example fluorescence) for each transcript relative to the unchanged housekeeping gene standards. Since the transcripts have various levels of expression and respectively their fold changes differ as well, instead of summing the raw expression values, they can be scaled and normalised between 0,1. Alternatively they can be weighted to allow important genes to carry greater effect. Then, for every sample the expression values of the signature's transcripts are summated, separately for the up- and down- regulated transcripts.

The total disease score incorporating the summated fluorescence of up- and down-regulated genes is calculated by adding the summated score of the down-regulated transcripts (after conversion to a positive number) to the summated score of the up-regulated transcripts, to give a single number composite expression score. This score maximally distinguishes the cases and controls and reflects the contribution of the up- and down- regulated transcripts to this distinction.

## Comparison of the disease risk score in cases and controls

The composite expression scores for patients and the comparator group may be compared, in order to derive the means and variance of the groups, from which statistical cut-offs are defined for accurate distinction of cases from controls. Using the disease subjects and comparator populations, sensitivities and specificities for the disease risk score may be calculated using, for example a Support Vector Machine and internal elastic net classification.

Disease risk score as employed herein is an indicator of the likelihood that patient has active TB when comparing their composite expression score to the comparator group's composite expression score.

## Development of the disease risk score into a simple clinical test for disease severity or disease risk prediction

The approach outlined above in which complex RNA expression signatures of disease or disease processes are converted into a single score which predicts disease risk can be used to develop simple, cheap and clinically applicable tests for disease diagnosis or risk prediction.

The procedure is as follows: For tests based on differential gene expression between cases and controls (or between different categories of cases such as severity), the up- and down- regulated transcripts identified as relevant may be printed onto a suitable solid surface such as microarray slide, bead, tube or well.

Up-regulated transcripts may be co-located separately from down-regulated transcripts either in separate wells or separate tubes. A panel of unchanged housekeeping genes may also be printed separately for normalisation of the results.

RNA recovered from individual patients using standard recovery and quantification methods (with or without amplification) is hybridised to the pools of up- and down-regulated transcripts and the unchanged housekeeping transcripts.

Control RNA is hybridised in parallel to the same pools of up- or down-regulated transcripts.

Total value, for example fluorescence for the subject sample and optionally the control sample is then read for up- and down- regulated transcripts and the results combined to give a composite expression score for patients and controls, which is/are then compared with a reference range of a suitable number of healthy controls or comparator subjects.

## Correcting the detected signal for the relative abundance of RNA species in the subject sample

The details above explain how a complex signature of many transcripts can be reduced to the minimum set that is maximally able to distinguish between patients and other phenotypes. For example, within the up-regulated transcript set, there will be some transcripts that have a total level of expression many fold lower than that of others. However, these transcripts may be highly discriminatory despite their overall low level of expression. The weighting derived from the elastic net coefficient can be included in the test, in a number of different ways. Firstly, the number of copies of individual transcripts included in the assay can be varied. Secondly, in order to ensure that the signal from rare, important transcripts are not swamped by that from transcripts expressed at a higher level, one option would be to select probes for a test that are neither overly strongly nor too weakly expressed, so that the contribution of multiple probes is maximised. Alternatively, it may be possible to adjust the signal from low-abundance transcripts by a scaling factor.

Whilst this can be done at the analysis stage using current transcriptomic technology as each signal is measured separately, in a simple colorimetric test only the total colour change will be measured, and it would not therefore be possible to scale the signal from selected transcripts. This problem can be circumnavigated by reversing the chemistry usually associated with arrays. In conventional array chemistry, the probes are coupled to a solid surface, and the amount of biotin-labelled, patient-derived target that binds is measured. Instead, we propose coupling the biotin-labelled cRNA derived from the patient to an avidin-coated surface, and then adding DNA probes coupled to a chromogenic enzyme via an adaptor system. At the design and manufacturing stage, probes for low-abundance but important transcripts are coupled to greater numbers, or more potent forms of the chromogenic enzyme, allowing the signal for these transcripts to be 'scaled-up' within the final single-channel colorimetric readout. This approach would be used to normalise the relative input from each probe in the up-regulated, down-regulated and housekeeping channels of the kit, so that each probe makes an appropriately weighted contribution to the final reading, which may take account of its discriminatory power, suggested by the weights of variable selection methods.

The detection system for measuring multiple up or down regulated genes may also be adapted to use rTPCR to detect the transcripts comprising the diagnostic signature, with summation of the separate pooled values for up and down regulated transcripts, or physical detection methods such as changes in electrical impedance. In this approach, the transcripts in question are printed on nanowire surfaces or within microfluidic cartridges, and binding of the corresponding ligand for each transcript is detected by changes in impedance or other physical detection system The present disclosure extends to a custom made chip comprising a minimal discriminatory gene set for diagnosis of active TB from other conditions, in particular those with similar symptoms, for example comprising at least 60-100% of the 42 genes listed in Table 3, and/or 60-100% of the 51 genes listed in Table 4.

In one embodiment the gene chip is a fluorescent gene chip that is to say the readout is fluorescence.

Fluorescence as employed herein refers to the emission of light by a substance that has absorbed light or other electromagnetic radiation.

Thus in an alternate embodiment the gene chip is a colorimetric gene chip, for example colorimetric gene chip uses microarray technology wherein avidin is used to attach enzymes such as peroxidase or other chromogenic substrates to the biotin probe currently used to attach fluorescent markers to DNA. The present disclosure extends to a microarray chip adapted to read by colorimetric analysis and adapted for the analysis of active TB infection in a child. The present disclosure also extends to use of a colorimetric chip to analyse a subject sample for active TB infection in children.

Colorimetric as employed herein refers to as assay wherein the output is in the human visible spectrum.

In an alternative described herein, a gene set indicative of active TB in children may be detected by physical detection methods including nanowire technology, changes in electrical impedance, or microfluidics.

The readout for the assay can be converted from a fluorescent readout as used in current microarray technology into a simple colorimetric format or one using physical detection methods such as changes in impedance, which can be read with minimal equipment. For example, this is achieved by utilising the Biotin currently used to attach fluorescent markers to DNA. Biotin has high affinity for avidin which can be used to attach enzymes such as peroxidase or other chromogenic substrates. This process will allow the quantity of cRNA binding to the target transcripts to be quantified using a chromogenic process rather than fluorescence. Simplified assays providing yes/no indications of disease status can then be developed by comparison of the colour intensity of the up- and down-regulated pools of transcripts with control colour standards. Similar approaches can enable detection of multiple gene signatures using physical methods such as changes in electrical impedance.

This aspect of the invention is likely to be particularly advantageous for use in remote or under-resourced settings or for rapid diagnosis in "near patient" tests. For example, places in Africa because the equipment required to read the chip is likely to be simpler.

Multiplex assay as employed herein refers to a type of assay that simultaneously measures several analytes (often dozens or more) in a single run/cycle of the assay. It is distinguished from procedures that measure one analyte at a time.

In one embodiment there is provided a bespoke gene chip for use in the method, in particular as described herein. Also herein described is a method of treating latent TB after diagnosis employing the method disclosed herein. Also herein described is a method of treating active TB after diagnosis employing the method disclosed herein.

Gene signature, gene set, disease signature, diagnostic signature and gene profile are used interchangeably throughout and should be interpreted to mean gene signature.

In the context of this specification "comprising" is to be interpreted as "including".

Aspects of the invention comprising certain elements are also intended to extend to alternative embodiments "consisting" or "consisting essentially" of the relevant elements.

Where technically appropriate, embodiments of the invention may be combined.

Embodiments are described herein as comprising certain features/elements. The disclosure also extends to separate embodiments consisting or consisting essentially of said features/elements.

Any embodiments specifically and explicitly recited herein may form the basis of a disclaimer either alone or in combination with one or more further embodiments.

## EXAMPLES

### Method

### Study sites and patient cohorts

In order to enable generalisation of our findings to African countries with differing prevalence of HIV, malaria, parasitic infections and differing environmental exposures that might affect transcriptional profiles, we established prospective cohorts of children undergoing investigation for TB in three sub-Saharan African countries with differing endemic diseases (Figure 2A).

We used a "discovery cohort" comprising children presenting with suspected TB to hospitals in South Africa and Malawi to identify RNA-transcript signatures associated with active TB. We then assessed performance of these signatures in an independent "validation cohort" of children presenting with suspected TB to hospitals in Kenya. The overall study design is shown in Figure 1B. Details of study sites are provided below and Figures 8a, b, c.

**Red Cross War Memorial Children's Hospital, Cape Town, South Africa (SA) -** SA has one of the highest paediatric TB incidence rates worldwide (981 per 100,000) (WHO Statistic 2011), as well as one of the most widespread national paediatric HIV epidemics (Kranzer et al 2011; WHO 2011). The Red Cross War Memorial Children's Hospital is a tertiary referral hospital in the Western Cape Province where malaria is not endemic. Despite >98% infant BCG vaccination coverage there is a high incidence of disseminated TB including TBM (van Well et al 2009).

**Queen Elizabeth Central Hospital, Blantyre, Malawi (MLW) -** is the tertiary public health facility for Blantyre, the major commercial centre of Malawi, where malaria, malnutrition and HIV are all endemic. Neonatal BCG vaccination coverage is 90% and countrywide HIV prevalence is 11% (National statistics office, Malawi 2005).

### Kilifi District Hospital (KDH) & Coast Provincial General Hospital (CPGH), Coast Province, Kenya -

Kenya is one of 22 'high TB burden' countries (Nelson et al 2004). KDH and CPGH serve a mixed rural and urban population in an area where malaria and malnutrition are endemic. HIV prevalence among women attending antenatal services is 4.4% and infant BCG vaccination coverage is 96% (Kenyan Bureau of national statistics 2010).

**Diagnostic process.** Children ≤14 years of age presenting to each hospital with suspected TB or a history of contact with an adult with TB were systematically investigated (Figure 1A) including chest radiography (CXR), HIV serology (plus HIV PCR for children <18 months old), complete blood count, Mantoux testing with 2 TU of PPD RT23 (SSI, Denmark), and commercial or previously validated in-house IGRA. Two spontaneous or induced sputum samples were examined by standard microscopy for acid fast bacilli (AFB) and cultured for mycobacteria. Isolation of MTB was confirmed by microscopic cording, MBT-64 lateral flow assays (Capilia®; TAUNS Laboratories, Inc., Numazu, Japan) and growth on p-nitrobenzoic acid (Malawi), plus specific PCR (SA and Kenya). The Xpert MTB/RIF real-time PCR assay was performed on respiratory samples in the Kenyan cohort. Bacterial cultures of blood, and CSF were undertaken and tissue samples (e.g. lymph node biopsies) sent for histology and culture where clinically indicated. Malaria was detected by routine Giemsa stained thick and thin film microscopy in Malawi and Kenya. All surviving children in whom a definitive diagnosis of TB had not been made underwent a further detailed clinical assessment at 3 months (SA and Malawi) or 1, 3 and 6 months (Kenya) post enrolment to confirm they remained TB-free.

**Case definitions:** We assigned individuals to one of five diagnostic groups based on HIV status and results at both enrolment and follow-up (Figure 2A). We defined active TB as isolation of MTB from a child with clinical features of TB; Other Diseases (OD) as the presence of a definitive alternative diagnosis to explain all the clinical features and/or no clinical deterioration at the end of follow-up in the absence of TB therapy, plus a negative TST and IGRA on enrolment (and a negative IGRA at three months where this was done); and LTBI as a positive TST and IGRA in a healthy child with no clinical or radiological evidence of TB who remained well at the end of follow-up (Figure 1A). Children who met none of the above definitions, in whom TB could be neither microbiologically confirmed nor confidently ruled out, were subsequently excluded from the study. All children (HIV-infected and -uninfected) with confirmed TB, a random selection of children with OD, and all HIV-uninfected children with LTBI were included in this analysis.

In an alternate analysis culture negative samples were included in the dataset for the validation cohort. Culture-confirmed TB was defined as isolation of MTB from a child with clinical features of TB; culture-negative TB as the presence of clinical and radiological features that prompted empiric treatment for TB but where mycobacterial culture confirmation was not obtained. Children with culture-negative TB were further categorized into "highly probable", "probable" and "possible" TB using a priori study definitions (Figure 2B). LTBI was defined as contact with a sputum smear positive TB case, positive TST and IGRA, and no evidence of TB at presentation or follow-up; OD as the presence of a definitive alternative diagnosis and/or no clinical deterioration on follow-up in the absence of TB therapy (Figure 2B).

Children with positive IGRAs were excluded from the OD group as self-limiting primary TB could not be excluded (Marais et al 2004). Assignments to diagnostic groups were made independently by two experienced clinicians (SA, AB), after reviewing investigations and any discrepancies adjudicated by a third clinician (BE).

**Ethical approval and consent**

The study was approved by the Research Ethics Committees of the University of Cape Town, South Africa (HREC REF 130/2007); the University of Malawi, College of Medicine, (COMREC P.12/07/599); the Liverpool School of Tropical Medicine (protocol number 08.12); Imperial College London (ICREC_9_1_1); and the Kenya Medical Research Institute (KEMRI/RES/7/3/1). Informed consent was obtained by trained health workers in local languages and parents or guardians provided either written consent or a thumb print.

**Oversight and conduct of the study**

Patients were recruited to the study by local health care workers. Assignment of patients to clinical groups was made by consensus of experienced clinicians at each site (independent of those managing the patient clinically) after review of the investigation results. Testing for HIV status was conducted after appropriate counselling. Isoniazid preventive therapy was administered to children under 5 years with LTBI according to national guidelines. Clinical data was anonymised and patient samples were identified only by study number. Microarrays were conducted by laboratory personnel blinded to assigned patient diagnostic groups. Statistical analysis was conducted only after the RNA expression data and clinical databases had been locked and deposited for independent verification.

**Peripheral whole blood RNA expression by microarray**

Whole blood was collected at the time of recruitment (either before or within 24 hours of commencing TB treatment in suspected cases) in PAXgene® blood RNA tubes (PreAnalytiX, Germany), frozen within 6 hours of collection and later extracted using PAXgene® blood RNA extraction kits (PreAnalytiX, Germany). RNA was shipped frozen to the Genome Institute of Singapore for analysis on HumanHT-12 v4 Expression BeadChips (Illumina).

Whole blood (2.5ml) was collected into PAXgene™ blood RNA tubes (PreAnalytiX, Germany), incubated for 2 hours, frozen at -20°C within 3 hours of collection, and then stored at -80°C. RNA was extracted using PAXgene™ blood RNA kits (PreAnalytiX, Germany) according to the manufacturer's instructions at one site (Cape Town) to minimize any sample handling bias. The integrity and yield of the total RNA was assessed using an Agilent 2100 Bioanalyser and a NanoDrop 1000 spectrophotometer respectively. Total RNA was then shipped to the Genome Institute of Singapore. After quantification and quality control, biotin-labelled cRNA was prepared using Illumina TotalPrep RNA Amplification kits (Applied Biosystems) from 500ng RNA. Labelled cRNA was hybridized overnight to Human HT-12 V4 Expression BeadChip arrays (Illumina). After washing, blocking and staining, the arrays were scanned using an Illumina BeadArray Reader according to the manufacturer's instructions. Using Genome Studio software the microarray images were inspected for artefacts and QC parameters were assessed. No arrays were excluded at this stage.

In an alternate analysis fourteen samples were excluded in total: 11 due to insufficient RNA after processing, 1 due to discrepant labelling, 2 removed at data QC in Principal Components Analysis (PCA).

**Statistical Analysis**

Expression data were analysed using *'R' Language and Environment for Statistical Computing (R)* 2.12.1. We used the South African/Malawi cohort to "discover" an RNA signature, and the Kenyan cohort for validation, reasoning that this approach would ensure that the signature was generalizable across different countries, with differing patterns of endemic disease. Recruited subjects from Malawi and SA were randomly assigned to a training cohort (80% of the subjects) that was used to identify significant transcripts distinguishing TB from LTBI and TB from OD, irrespective of HIV status or geographic location, and the results tested on the remaining 20% (Test cohort).

To detect transcripts that were differentially expressed between TB cases and comparator groups, a linear model was fitted and moderated t-statistics calculated for each transcript with correction for false discovery using Benjamini and Hochberg's method. To identify the smallest number of transcripts distinguishing TB from comparator groups, significantly differentially expressed (SDE) transcripts in the discovery cohort with a $\log_2$ fold change (FC) >0.5 were subjected to variable selection using elastic net. These minimal transcript selected sets for TB vs LTBI, and TB vs OD were evaluated in the test cohort (Malawi and SA samples) and then finally validated on the independent Kenyan cohort.

Mean raw intensity values for each probe were corrected for local background intensities and a robust spline normalisation (combining quantile normalisation and spline interpolation) was applied to each array. Expression values were transformed to a logarithmic scale (base 2), and for each probe. Differential expression between patient groups was identified by fitting a linear model to each transcript using LIMMA[2]. P-values were adjusted using the method of Benjamini and Hochberg. Transcripts with log FC >0.5 were taken forward to variable selection with elastic net. This threshold was chosen in order to ensure that differential expression for selected variables could be distinguished using the resolution of qtPCR. The $\alpha$ and $\lambda$ parameters of elastic net, which control the size of the selected model, were optimized via ten-fold cross-validation (CV). The weights assigned by elastic net to the trained model were used within a linear regression model to classify samples in the test set.

In an alternate analysis Mean raw intensity values for each probe were corrected for local background intensities and a robust spline normalisation (combining quantile normalisation and spline interpolation) was applied to each array. Expression values were transformed to a logarithmic scale (base 2). PCA was used as part of the quality control process of the arrays before the split into 80%-20% for the identification of signatures. PCA is an approach that allowed us to summarize our data and reduce the dimensionality (536 arrays x 48,000 probes, down to 536 arrays x no of principal components) in order to explore variance in the expression level. RNA expression profiles of most children in the discovery cohort clustered together on PCA; two outlying samples were removed from the analysis (Figure 5). At the two first principal components there was no variance introduced because of location or HIV status of the samples (Figure 5). Using the 2-dimensional equivalent of the t-statistic, the Hotelling test, we removed two samples before the analysis (categorized as TB/HIV+ and OD/HIV+ from Malawi). The samples were divided into a training set ($n_{TB}$=87 $n_{OD}$=134 HIV+/-; $n_{TB}$=56 $n_{OD}$= 82 HIV-, $n_{TB}$=31 $n_{OD}$=52 HIV+, $n_{LTBI}$=43 HIV-) and test set ($n_{TB}$=23 $n_{OD}$=34 HIV+/-; $n_{TB}$=14 $n_{OD}$= 21 HIV-, $n_{TB}$=9 $n_{OD}$=13 HIV+, $n_{LTBI}$=11 HIV-). Using the training set, we identified the transcripts that were differentially expressed between patient groups with $|\log_2$ FC| >0.5, which were taken forward to variable selection with elastic net. This threshold was chosen in order to ensure that differential expression for selected variables could be distinguished using the resolution of qtPCR. The **a** and $\lambda$ parameters of elastic net, which control the size of the selected model, were optimized via ten-fold cross-validation (CV). The weights assigned by elastic net to the trained model were used within a linear regression model to classify samples in the test set.

## A simplified method for identifying individual patient's risk of active TB

Current whole genome array-based technologies are not well suited for use in resource poor settings as they are costly and require sophisticated technology as well as bioinformatics expertise. We therefore developed a method for translation of multiple transcript RNA signatures into a disease risk score, which could form the basis of a simple, low cost, diagnostic test requiring basic laboratory facilities and minimal bioinformatics analysis.

For each individual, we calculated the disease risk score (DRS) using the minimal transcript selected sets for pTB vs. pLTBI and pTB vs. pOD. The score is derived by adding the total intensity at up-regulated transcripts, and subtracting the total intensity at all down-regulated transcripts. The sensitivity and specificity of this score in disease classification was evaluated on the SA/Malawi 20% cohort and the independent Kenyan validation cohort.

$$Threshold = \frac{\left(\dfrac{\mu_1}{\sigma_1} + \dfrac{\mu_2}{\sigma_2}\right)}{\left(\dfrac{1}{\sigma_1} + \dfrac{1}{\sigma_2}\right)}$$

Where $\mu_n$ is the mean of comparator group $n$, and $\sigma_n$ is the standard deviation of comparator group n. The performance of the simplified risk score was then evaluated in our cohort as well as the independent datasets.

*Disease risk score*

For each individual, we calculated the disease risk score using the minimal transcript selected sets for pTB vs. pLTBI and pTB vs. pOD. The score is based on subtracting the summed intensities of the down-regulated transcripts from the summed intensities of the up-regulated transcripts. The disease risk score for an individual is:

$$Disease\ Risk\ Score^i = \sum_{k=0}^{n} expr.value_k^i - \sum_{l=0}^{m} expr.value_l^i \qquad (1)$$

where: **n** the number of up-regulated number of probes in the signature in disease of interest (TB) compared to comparator group(s).

**m** the number of down-regulated number of probes in the signature in disease of interest (TB) compared to comparator group(s).

The threshold for the classification was calculated as the weighted average of risk score within each class, with weights given as inverse of the standard deviation of the score within each class (1/sd1 and 1/sd2 respectively). The threshold for the classification between group u and v is shown below:

$$threshold(u,v) = \frac{\frac{\mu_u}{\sigma_u} + \frac{\mu_v}{\sigma_v}}{\frac{1}{\sigma_u} + \frac{1}{\sigma_v}} \qquad (2)$$

where: $\mu$ average of the disease risk score in the group.

$\sigma$ standard deviation of the disease risk score in the group.

To calculate the indeterminate zone, we calculated the lower and upper threshold which were calculated as the weighted average with weights given by w/sd1, (1-w)/sd2 respectively for variable 0.5 < w <= 1. When w = 0.5 its equivalent formula to main threshold. ROCs were generated using pROC5.

### Evaluation of performance of DRS and comparison with Xpert MTB/RIF

We compared the performance of both DRS and Xpert MTB/RIF in the culture-confirmed, "highly probable", "probable" and "possible" culture-negative TB groups separately. In each case we used the same OD group as the TB-negative comparator group to calculate test specificity. In order to obtain more realistic estimates of the test sensitivity across the culture-negative TB categories, we recognized that each category is a mixture of "actual" TB cases and OD clinically confused with TB. We therefore, modelled the observed true-positive rate (TPR) as a function of the unknown actual TPR, the false-positive rate estimated from the OD group, and the prevalence of TB (Eq. 3), from which we calculated a corrected Receiver Operator Characteristic (ROC) curve and estimates of 'effective' sensitivity in each category. As the prevalence of TB in each category is unknown, we investigated a range of prevalence of 70%-90%, 40%-60% and 30%-50% for "highly probable", "probable" and "possible" TB respectively and also present unadjusted results which are equivalent to assuming a TB prevalence of 100% in each category.

### Analysis of validation datasets

For validation of the performance of the disease risk score based on the pTB vs. pLTBI 42 transcript signature and pTB vs. pOD 51 transcript signature, we used the an independent cohort recruited from Kilifi, Kenya. The microarray analysis was as previously described but the cohort from Kenya was processed independently from the other two and normalised separately.

In an alternate analysis the Kenyan validation cohort contained culture-negative patients. The Kenyan validation cohort ($n_{TB}$=35 $n_{OD}$=55 HIV+/-; $n_{TB}$=25 $n_{OD}$= 29 HIV-, $n_{TB}$=10 $n_{OD}$=26 HIV+, $n_{LTBI}$=14 HIV-) was not included in the initial analysis and derivation of the signatures. The microarray analysis for the Kenyan validation cohort was done as previously described, but the raw microarray data were pre-processed (background subtracted and normalized) separately from the discovery cohort. We then calculated the disease risk scores, based on the signatures derived in the discovery cohort, for the samples of the Kenyan cohort to evaluate their performance in an independent validation cohort.

### Calculation of effective sensitivity in culture-negative TB groups

Application of a classifier, such as DRS, to a culture-negative TB group results in an observed estimate of the true-positive rate ($TPR_{obs}$), which is the proportion of all observed 'positives' ($P_{obs}$) scored as 'true' by the classifier. However, these observed positives are in fact a mixture of actual true TB and false TB (i.e. OD), hence

$$TPR_{obs} = \frac{TP_{obs}}{P_{obs}} = \frac{TP_{actual} + FP_{actual}}{P_{actual} + F_{actual}} = \frac{TPR_{effective} * P_{actual} + FPR_{effective} * F_{actual}}{P_{actual} + F_{actual}}$$

$$= TPR_{effective} * \frac{P_{actual}}{P_{actual} + F_{actual}} + FPR_{effective} * \frac{F_{actual}}{P_{actual} + F_{actual}}$$

$$= TPR_{effective} * \Pr(TB) + FPR_{effective} * (1 - \Pr(TB)) \qquad (3)$$

where: $F_{actual}$ is the number of OD and Pr(TB) is the prevalence of actual TB and in the group under consideration. $FPR_{effective}$ is the false-positive rate at which OD are falsely called TB by the classifier, and can be estimated using the OD group. We can re-arrange equation (3) to obtain a formula for the effective TPR in terms of the group prevalence and the FPR estimated from the OD group:

$$TPR_{effective} = \frac{TPR_{obs} - FPR_{actual} * (1 - \Pr(TB))}{\Pr(TB)} \qquad (4)$$

**Positive and negative predictive value for combined culture positive and culture negative TB**

We calculated the positive and negative predictive value (PPV and NPV) as a function of specificity sensitivity and prevalence according to the following formulae:

$$NPV = \frac{specificity * (1 - prevalence)}{specificity * (1 - prevalence) + (1 - sensitivity) * prevalence} \qquad (5)$$

$$PPV = \frac{sensitivity * prevalence)}{sensitivity * prevalence + (1 - sensitivity) * (1 - prevalence)} \qquad (6)$$

Given the dependency of NPV/PPV on test sensitivity, specificity and prevalence, it is important to provide estimates of these values specific to scenarios in which such a diagnostic test would be applied. We have calculated these values for a scenario in which a child presents to a clinic with symptoms consistent with TB, and thus we use the specificity as reported in Table 2a for the HIV-infected and -uninfected combined other disease group from the Kenyan validation set.

We use a test sensitivity estimate derived on the combined culture-positive and culture-negative TB groups. We estimated this as a weighted average of the 'effective' sensitivity in the culture-confirmed, "highly probable" (HP), "probable" (Pr) and "possible" (Pos) TB, with the weights given by the proportion of samples in the Kenyan prospective study which were assigned to each of these groups. The effective sensitivity in each subgroup was calculated using equation 4, based on the same range of assumptions on the prevalence of 'actual' TB in each group used to calculate effective sensitivities in Table 2C. In more detail, the scenarios considered are:

- A: TB prevalence in: culture confirmed = 100%; HP TB = 70%; Pr TB =40%; Pos TB = 30%

- B: TB prevalence in: culture confirmed = 100%; HP TB = 80%; Pr TB =50%; Pos TB = 40%

- C: TB prevalence in: culture confirmed = 100%; HP TB = 90%; Pr TB =60%; Pos TB = 50%

Also recognizing that the prevalence of TB among the population tested will depend on the operational clinical strategy for TB screening, we used a range of estimates for TB population prevalence in the patients screened (10%, 30% and 50%).

- 10%: reflects the prevalence of TB in the Kenyan cohort

- 30%: reflects the prevalence of TB in the South Africa and Malawi recruitment

- 50%: reflects a scenario that clinicians would do prior filtering or combining with another test

The proportion of proven TB amongst patients suspected of having TB varies depending on the strategy for TB investigation. In South Africa and Malawi, patients were investigated for TB if the clinicians responsible for the child's care considered TB to be included in the differential diagnosis. In Kenya, a systematic screening process was undertaken for all children with cough, fever or weight loss of > 2 weeks duration. The difference in approach resulted in different proportions of TB cases, with the broad criteria used in Kenya being reflected in a lower proportion of TB. As our research setting actively sought to identify TB cases, it is likely that in a non-research setting in typical African hospitals, patients selected to undergo investigation for TB may make up a higher proportion of those investigated, hence our exploration of 50% prevalence scenario.

## Results

We recruited 157 patients to the South African, 189 to the Malawian and 106 to the Kenyan cohort after screening a total of 3876 children in the three sites. After technical failures, 436 samples remained for analysis (Figure 2A; Table 1A). Clinical and demographic features of these patients are shown in Table 1a. The spectrum of diseases in the OD cohorts included a range of conditions, many with similar clinical manifestations to TB (Table 1B).

Principal component analysis (Figure 5) showed that the patients in the discovery cohort had similar RNA expression profiles, although two samples were outliers on the PCA analysis and were removed from the analysis.

In the alternate dataset the validation cohort contained culture negative patients:

After screening and investigating 1356 children for symptoms of TB in South Africa and Malawi, we included 157 patients from South Africa and 189 from Malawi in the RNA expression studies. 114 children had culture confirmed TB, 175 had OD where TB was excluded and 57 had LTBI. The discovery cohort included only "gold standard" culture-confirmed TB cases and excluded patients where the diagnosis of TB could not be confidently assigned or excluded. Details of recruitment are given herein, Figure 8a, b. Patient clinical details are summarized in Table 1C, and Table 1B.

## Identification and validation of minimal transcript-sets

To find minimal transcript sets required to discriminate TB from other groups we applied the variable selection algorithm elastic netto the training cohort. A 42 transcript model was identified for discriminating pTB from pLTBI (Table 3), whilst a 51 transcript model was identified for discriminating pTB from pOD (Table 4) which were also found to distinguish pTB in the Kenyan validation cohort.

After separating the discovery cohort into training and test sets (80% and 20% of the samples) we identified 409 transcripts in the training set significantly differentially expressed (SDE) between TB and OD, and 3434 transcripts between TB and LTBI. After variable selection to identify the smallest number of transcripts distinguishing TB/OD and TB/LTBI, we identified 51 and 42 transcripts respectively (list of transcripts in Table S2a, b). These minimal transcript sets were used to generate a DRS for each patient which in the test set, distinguished TB from OD and LTBI with sensitivity/specificity of 78/74% and 96/91% respectively (Table 3, Table 4, Figure 4).

## Evaluation of a simplified disease risk score for TB

To evaluate the feasibility of using a simplified diagnostic test based on our transcript sets for TB diagnosis in low resource settings, we applied the simplified risk score to the SA/Malawi discovery cohort, and then validated the results in the independent Kenyan cohort. The disease risk score discriminated pTB from pLTBI with sensitivity/specificity in the independent Kenyan cohort of 94%/100%, and pTB from pOD with sensitivity/specificity 84%/83% (Figure 4, Table 2). Remarkably, the performance of the score was as good in the HIV-infected patients as in those HIV-uninfected (Table 2, Figure 6).

## Validation of DRS in the Kenyan cohort (culture negative included) and comparison with Xpert-MTB/RIF

1599 children presenting to hospital in Kenya met the study inclusion criteria and 1471 were investigated for TB. We included 148 children in a nested case-control study of RNA expression that included both culture-confirmed and culture-negative ("highly probable", "probable" and "possible") TB groups. We included all culture-confirmed TB and LTBI patients with adequate RNA samples (35 and 14 respectively), 44 culture-negative TB patients and 55 randomly selected OD cases. Clinical features are summarised in Table 1b. Among the culture-negative TB group, we included patients in each sub-category in proportion to the expected number of "actual" TB cases in each category (8 "highly probable", 19 "probable" and 17 "possible" TB, see Table 6). Patients included in the array study had similar clinical features to those not included; a "history of close TB contact" in the "probable" TB and OD groups was the only significant difference between arrayed and non-arrayed TB patients (Table 7a,b). There was no association between TB contact and a positive DRS (p=0.104).

The DRS discriminated culture-confirmed TB from OD in both HIV-uninfected and -infected cases with sensitivity of 83% and specificity of 84% (Figure 7, Table 3). The DRS also distinguished TB from LTBI (sensitivity 94%, specificity 100%; Figure 4, Table 5). Among the culture-negative cases treated for TB, the DRS identified 63% of "highly probable", 42% of "probable" TB, and 35% of "possible" TB cases as having TB. After adjusting for the estimated prevalence of "actual" TB in each group, DRS had an effective sensitivity of 68 - 82%, 59 - 81%, 54 - 80% among "highly probable", "probable" and "possible" TB cases respectively (Table 4). The sensitivity of the DRS was higher than Xpert MTB/RIF in all TB categories (Figure 7, Table 4) with Xpert MTB/RIF sensitivities in the same culture confirmed, "highly probable", "probable" and "possible" categories being 54%, 25%, 5% and 0%. Despite the lower sensitivity, Xpert MTB/RIF was highly specific (100%).

To explore how the DRS for TB/OD might contribute to TB diagnosis in clinical practice, we evaluated its positive and negative predictive value (PPV, NPV) in a setting with 10% TB prevalence (as observed in the validation cohort), a 30% TB prevalence setting (as observed in the discovery cohort), as well as a 50% TB prevalence setting, which might reflect a non-research setting where more targeted clinical filtering criteria are applied. We also included a range of estimates of actual TB in the culture-negative TB group. NPV was consistently high in all three cohorts. As expected PPV was higher when more targeted clinical criteria were used to select those tested (Table 8).

## Discussion

We have identified host blood transcriptomic signatures that distinguish pTB from pLTBI and from a wide range of other diseases frequently clinically indistinguishable from TB in African children, irrespective of their HIV status. Children with TB were distinguished from those with LTBI and OD with 42 and 51 transcripts respectively. The signatures allowed nearly complete discrimination of TB from LTBI (with sensitivity and specificity of over 94%), and also distinguished TB from OD with sensitivity and specificity of over 80% irrespective of HIV status. Our findings extend previous studies on RNA expression in adult TB (Berry et al 2010; Jacobsen et al 2007; Lesho et al 2011; Lu et al 2011; Maertzdorf et al 2012; Mistry et al 2007), which suggested existence of a unique TB signature, and our companion study (under review) shows that relatively small numbers of transcripts can distinguish TB in adults from other diseases. Our study is the first identifying a TB-specific signature in both HIV-infected and -uninfected children with TB. The relatively small number of transcripts in our signatures suggests the potential to use RNA expression profiling on a single peripheral blood sample as a clinical diagnostic tool.

The major challenge in evaluating new biomarkers of childhood TB is the lack of a 'gold standard' against which to evaluate them, as microbiological confirmation is achieved in a minority of cases commenced on TB treatment. It is generally accepted that clinical diagnostic scores over-diagnose TB, but the extent of over-diagnosis is unknown. Conversely, missed diagnosis of TB inadvertently treated as OD is common. To address this challenge we first evaluated performance of our signatures and DRS against the 'gold standard' of culture-confirmed TB, and then explored their performance in the culture-negative group for which no 'gold standard' is available. The approach we developed in which estimates of the "true" proportion of TB in "highly probable", "probable" and "possible" TB categories are used to calculate an "effective" sensitivity of the DRS optimizes evaluation of biomarkers in culture-negative TB. The gradient in DRS performance observed in the culture-negative TB groups is consistent with the differing degrees of diagnostic certainty in each group. Our findings suggest that the DRS provides a better estimate of actual prevalence in each category, and indicates considerable over-diagnosis and over-treatment of childhood TB even in a research setting where more sophisticated diagnostic tools were available than in most African hospitals.

In order to establish the potential role of our RNA-based approach, we compared our DRS with the best available diagnostic methods including both culture and detection of mycobacterial DNA using the Xpert MTB/RIF assay. Although Xpert is highly specific, our study confirms others showing that sensitivity in childhood TB is limited. Our DRS identified a higher proportion of culture-confirmed TB cases, and a greater proportion of culture-negative cases than Xpert. Although some culture-confirmed TB cases were "missed" by our score, improvement in sensitivity of the method may be achieved in future by weighting the transcripts in the signature, by inclusion of additional transcripts, or by incorporating DRS into an investigation protocol where patients with a negative DRS who remain ill undergo additional investigations.

Our simplified risk score, which enables multi-transcript RNA signatures to be translated into a single number - the individual risk score - suggests that development of a simple test for childhood TB is feasible, if a point of care test can be developed using less complex technology than microarray.

Our signatures and the disease risk score accurately distinguish the majority of patients who have TB from those with OD and/or LTBI in whom TB is excluded.

Our study provides proof of principle that diagnosis of active TB in African countries affected by the HIV/TB epidemic is feasible using RNA expression on peripheral blood.

**Table 1A.** Clinical and diagnostic features of South Africa, Malawi and Kenya cohorts with active tuberculosis (TB), latent TB Infection (LTBI) or Other Diseases (OD).

| Group | TB/HIV- | | | TB/HIV+ | | | LTBI/HIV- | | | OD/HIV+ | | | OD/HIV- | | |
|---|---|---|---|---|---|---|---|---|---|---|---|---|---|---|---|
| Location | SA | Malawi | Kenya | SA | Malawi | Kenya | SA | Malawi | Kenya | SA | Malawi | Kenya | SA | Malawi | Kenya |
| No. children | 50 | 22 | 25 | 23 | 20 | 10 | 4 | 53 | 14 | 30 | 41 | 26 | 50 | 55 | 29 |
| Median age, months (IQR) | 29 (15; 96) | 99 (55; 134) | 37 (12; 106) | 62 (43; 102) | 96 (52; 140) | 101 (53; 127) | 35 (29; 50) | 48 (23; 91) | 33 (18; 43) | 23 (13; 66) | 96 (47; 156) | 39 (16; 78) | 15 (11; 25) | 47 (20; 104) | 20 (11; 82) |
| Male (%) | 62 | 55 | 52 | 65 | 50 | 60 | 50 | 53 | 50 | 50 | 49 | 73 | 64 | 62 | 69 |
| Median WAZ score (IQR) | -1.5 (-2.3; -0.4) | -2.7 (-3.4; -0.9) | -2.4 (-3.4; -1.0) | -1.78 (-2.5; -1.1) | -3.2 (-3.7; -3.0) | -3.6 (-4.3; -2.7) | -0.62 (-1.8; 0.4) | -1.1 (-2.1; 0.0) | -1.6 (-2.1; -1.2) | -1.2 (-3.0; -0.6) | -2.3 (-3,9; -1.0) | -3.1 (-4.4; -1.7) | -1.2 (-1.9; -0.0) | -1.1 (-2.2; -0.5) | -2.7 (-3.8; -2.2) |
| BCG vaccinated (%) | 42/45 (93%) | 19/19 (100%) | 24/25 (96%) | 17/18 (94%) | 17/17 (100%) | 9/10 (90%) | 4/4 (100%) | 51/51 (100%) | 11/14 (79%) | 25/25 (100%) | 35/35 (100%) | 22/25 (88%) | 47/48 (97.92) | 52/53 (98%) | 26/29 (90%) |
| Median CD4 count/mm$^3$ (IQR) | NA | NA | NA | 640 (169; 812) | 418 (278; 762) | ND | NA | NA | NA | 531 (315; 805) | 347 (145-595) | ND | NA | NA | NA |
| Median CD4 % (IQR) | NA | NA | NA | 18.7 (12.6; 26.3) | ND | ND | NA | NA | NA | 20.9 (11.6; 22.4) | ND | ND | NA | NA | NA |
| TST positive* | 38/50 (76%) | 16/22 (73%) | 17/25 (68%) | 10/20 (50%) | 8/20 (40%) | 3/10 (30%) | 4/4 (100%) | 53/53 (100%) | 14/14 (100%) | 0/30 (0%) | 0/40 (0%) | 0/23 (0%) | 0/50 (0%) | 0/50 (0%) | 0/29 (0%) |
| IGRA positive | 39/50 (78%) | 18/22 (82%) | 14/20 (70%) | 17/22 (77%) | 14/20 (70%) | 4/8 (50%) | 4/4 (100%) | 53/53 (100%) | 14/14 (100%) | 0/30 (0%) | 0/40 (0%) | 0/23 (0%) | 0/50 (0%) | 0/50 (0%) | 0/29 (0%) |
| Malaria positive | ND | 1/20 (5%) | 0/25 (0%) | ND | 1/19 (5.3%) | 0/10 (0%) | ND | 0/52 (0%) | 0/14 (0%) | ND | 0/41 (0%) | 1/26 (4%) | ND | 6/55 (11%) | 4/29 (14%) |

SA = South Africa, TB = active TB, LTBI = latent TB infection, OD = other diseases (see below), HIV- = HIV-uninfected, HIV+ = HIV-infected, IQR= interquartile range, WAZ = weight-for-age z-score, TST = tuberculin skin test, IGRA= interferon gamma release assay, ND= not done, NA= not applicable.

* A positive TST was defined according to WHO guidelines as an induration of ≥10mm; or ≥ 5mm in children with HIV infection or severe malnutrition.

EP 2 914 740 B1

Table 1B. Major clinical diagnoses in the 'Other Diseases' groups from each of the study sites.

| Group | HIV-infected | | | HIV-uninfected | | | |
|---|---|---|---|---|---|---|---|
| Location | SA | Malawi | Kenya | SA | Malawi | Kenya | Total |
| Pneumonia* | 24 | 15 | 15 | 30 | 17 | 18 | 119 |
| Bronchiectasis/chronic lung disease | 2 | 7 | - | - | 1 | - | 10 |
| Lymphocytic Interstitial Pneumonitis | - | 2 | - | - | - | - | 2 |
| Upper respiratory tract infection (URTI) | - | - | - | 11 | - | - | 10 |
| Inflammatory bone and joint diseases | - | 1 | - | - | 8 | 2 | 11 |
| Bacterial soft tissue infection | - | 5 | - | - | 16 | - | 21 |
| Gastroenteritis | 2 | - | - | 5 | - | - | 7 |
| Infection at ≥2 sites+ | 2 | - | 1 | 2 | - | - | 5 |
| Sepsis without a focus⇑$ | - | - | 3 | - | - | 1 | 4 |
| Kaposi Sarcoma (KS)% | - | 7 | - | - | - | - | 7 |
| Other malignancy‡ | - | - | - | - | 5 | 1 | 6 |
| Malaria + severe malnutrition | - | - | - | - | 1 | 3 | 4 |
| Primary diagnosis of severe malnutrition | - | 1 | 4 | - | 1 | 3 | 9 |
| Other# | - | 2 | 3 | 2 | 6 | 1 | 14 |
| Total | 30 | 40 | 26 | 50 | 55 | 29 | 230 |

* Includes 10 with pneumonia and bacteremia; one of whom also had empyema.

+ Includes pneumonia + gastroenteritis (2); pneumonia + urinary tract infection + gastroenteritis (1); *E. coli* bacteremia + malaria (1); osteomyelitis + malaria (1); bacterial meningitis + URTI (1); gastroenteritis + URTI (1).

⇑ Includes bacteraemic sepsis (3) and septic shock without bacteremia (1).

$ These are children who had a primary diagnosis of severe malnutrition; many of the other children in the OD group also had severe malnutrition in addition to the diagnoses listed.

% Includes one child with KS and septicemia.

‡ All histologically confirmed. Includes Burkitt's lymphoma (2); rhabdomyosarcoma (1); non-Hodgkin's lymphoma (1); and metastatic carcinoma of uncertain origin (1).

# Includes abscess + bacteremia (1); meningitis (1); empyema (1); severe anemia (1); and one child with severe malnutrition and a febrile illness of uncertain aetiology which resolved without TB treatment.

**Table 1C. Clinical features of children in the South Africa/Malawi discovery cohort**

| | TB / HIV- | | TB / HIV+[†] | | LTBI / HIV- | | OD / HIV+[†] | | OD / HIV- | |
|---|---|---|---|---|---|---|---|---|---|---|
| Location | SA | Malawi | SA | Malawi | SA | Malawi | SA | Malawi | SA | Malawi |
| No. children | 50 | 22 | 23 | 19 | 4 | 53 | 30 | 40 | 50 | 55 |
| Median age, months (IQR) | 29 (15; 96) | 99 (55; 134) | 62 (43; 102) | 89 (51; 130) | 35 (29; 50) | 48 (23; 91) | 23 (13; 66) | 97 (51; 156) | 15 (11; 25) | 47 (20; 104) |
| Male (%) | 62 | 55 | 65 | 58 | 50 | 53 | 50 | 49 | 64 | 62 |
| Median WAZ score (IQR) | -1.5 (-2.3; -0.4) | -2.7 (-3.4; -0.9) | -1.78 (-2.5; -1.1) | -2.9 (-3.2; -2.3) | -0.62 (-1.8; 0.4) | -1.1 (-2.1; 0.0) | -1.2 (-3.0; -0.6) | -2.2 (-2,8; -1.7) | -1.2 (-1.9; -0.0) | -1.1 (-2.2; -0.5) |
| BCG vaccinated (%) | 42/45 (93%) | 19/19 (100%) | 17/18 (94%) | 17/17 (100%) | 4/4 (100%) | 51/51 (100%) | 25/25 (100%) | 34/34 (100%) | 47/48 (97.92) | 52/53 (98%) |
| Median CD4 count/mm$^3$ (IQR) | NA | NA | 640 (169; 812) | 418 (278; 762) | NA | NA | 531 (315; 805) | 349 (141; 611) | NA | NA |
| Median % CD4 count (IQR) | NA | NA | 18.7 (12.6; 26.3) | ND | NA | NA | 20.9 (11.6; 22.4) | ND | NA | NA |
| TST positive* | 38/50 (76%) | 16/22 (73%) | 10/20 (50%) | 7/19 (37%) | 4/4 (100%) | 53/53 (100%) | 0/30 (0%) | 0/40 (0%) | 0/50 (0%) | 0/50 (0%) |
| IGRA positive | 39/50 (78%) | 18/22 (82%) | 17/22 (77%) | 13/19 (68%) | 4/4 (100%) | 53/53 (100%) | 0/30 (0%) | 0/40 (0%) | 0/50 (0%) | 0/50 (0%) |

SA = South Africa, TB = active TB, LTBI = latent TB infection, OD = other diseases (see below), HIV- = HIV-uninfected, HIV+ = HIV-infected, IQR= interquartile range, WAZ = weight-for-age z-score, TST = tuberculin skin test, IGRA= interferon gamma release assay, ND= not done, NA= not applicable. [†]12 of the HIV+ children in Malawi were on ART and 0 of the children from South Africa. *A positive TST was defined according to WHO guidelines as an induration of ≥10mum; or ≥ 5mm in children with HIV infection or severe malnutrition with 2 TU of PPD RT23 (SSI, Denmark). Discrepancies in total number of children in each category and number with a visible BCG scar denote cases in whom it was difficult to determine whether a scar was present.

**Table 1D. Clinical features of children in the Kenyan validation cohort[1].**

| | TB / HIV- | TB / HIV+[†] | Culture-negative TB / HIV- | Culture-negative TB / HIV+[†] | LTBI / HIV- | OD / HIV- | OD / HIV+[†] |
|---|---|---|---|---|---|---|---|
| No. children | 25 | 10 | 27 | 17 | 14 | 29 | 26 |
| Median age, months (IQR) | 37 (12; 106) | 101 (53; 127) | 22 (12; 40) | 32 (18; 60) | 33 (18; 43) | 20 (11; 82) | 39(16; 78) |
| Male (%) | 13 (52%) | 6 (60%) | 15 (56%) | 10 (59%) | 7 (50%) | 20 (69%) | 19 (73%) |
| Median WAZ score (IQR) | -2.4 (-3.4; -1.0) | -3.6 (-4.3; -2.7) | -2.5 (-3.6; -1.6) | -3.5 (-4.5; -3.0) | -1.6 (-2.1; -1.2) | -2.7 (-3.8;-2.2) | -3.1 (-4.4; -1.7) |
| BCG vaccinated (%) | 24 (96%) | 9 (90%) | 23 (85%) | 16 (94%) | 11 (79%) | 26 (90%) | 22/25 (88%) |
| Close TB contact history | 14 (56%) | 8 (80%) | 14 (52%) | 7 (41%) | 14 (100%) | 1 (3%) | 4 (15%) |
| TST positive* | 17 (68%) | 3 (30%) | 13 (48%) | 2 (12%) | 14 (100%) | 0 (0%) | 0 (0%) |
| IGRA positive | 7/10 (70%) | 2/5 (20%) | 9/14 (64%) | 3/10 (30%) | 14 (100%) | 0/24 (0%) | 0/21 (0%) |
| Persistent cough >2 weeks | 15 (60%) | 8 (80%) | 14 (52%) | 14 (82%) | 0 (0%) | 9 (31%) | 13 (50%) |
| Persistent fever >2 weeks | 15 (60%) | 7 (70%) | 9 (33%) | 9 (53%) | 0 (0%) | 11 (38%) | 9 (35%) |
| Night sweats >2 weeks | 9 (36%) | 6 (60%) | 10 (37%) | 2 (12%) | 0 (0%) | 3 (10%) | 6 (23%) |
| Weight loss or failure to thrive | 16 (64%) | 9 (90%) | 18 (67%) | 13 (76%) | 1 (7%) | 24 (83%) | 19 (73%) |
| CXR features of TB | 19 (76%) | 9 (90%) | 17 (63%) | 12 (71%) | 0 (0%) | 8 (26%) | 11 (42%) |
| TB culture positive | 25 (100%)[a] | 10 (100%)[a] | 0 (0%) | 0 (0%) | - | 0 (0%) | 0 (0%) |

TB = confirmed TB, LTBI = latent TB infection, OD = other diseases (see below), HIV- = HIV-uninfected, HIV+ = HIV-infected, IQR= interquartile range, WAZ = weight-for-age z-score, TST = tuberculin skin test, IGRA= interferon gamma release assay, ND= not done, NA= not applicable. Where data on a particular feature were not available for all patients in a group, the denominator for which data were available is indicated. A positive TST was defined according to WHO guidelines as an induration of $\geq$10mm; or $\geq$ 5mm in children with HIV infection or severe malnutrition. [†]16 of the HIV+ children were on ART. [a]6 AFB smear positive.
[1]See reference Graham et al 2012

**Table 2A.** Diagnostic performance of the TB/LTBI and TB/OD disease risk scores.

| | South Africa/Malawi Training Cohort | | | South Africa/Malawi Testing Cohort | | | Kenya - Independent Validation Cohort | | |
|---|---|---|---|---|---|---|---|---|---|
| | HIV- & HIV+ | HIV- | HIV+ | HIV-& HIV+ | HIV- | HIV+ | HIV- & HIV+ | HIV- | HIV+ |
| TB vs. latent TB infection (42 TB/LTBI transcript signature) | | | | | | | | | |
| Area under ROC (95% CI) | 98.4 (96.3 - 99.8) | - | - | 98.4 (94.5 - 100.0) | - | - | 100.0 (100.0 - 100.0) | - | - |
| Sensitivity, % (95% CI) | 95.4 (90.8 - 98.9) | - | - | 95.7 (87.0 - 100.0) | - | - | 94.3 (85.7 - 100.0) | - | - |
| Specificity, % (95% CI) | 97.7 (93.0 - 100.0) | - | - | 90.9 (72.7 - 100.0) | - | - | 100.0 (100.0 - 100.0) | - | - |
| TB vs. Other Diseases (51 TB/OD transcript signature) | | | | | | | | | |
| Area under ROC (95% CI) | 95.6 (93.3 - 97.7) | 96.9 (94.2 - 98.9) | 93.0 (87.5 - 97.2) | 86.2 (77.1 - 94.0) | 88.4 (75.9 - 97.6) | 84.6 (64.0 - 96.6) | 89.0 (82.3 - 94.9) | 85.7 (75.0 - 94.4) | 93.9 (83.9 - 100.0) |
| Sensitivity, % (95% CI) | 88.5 (81.6 - 94.3) | 92.9 (85.7 - 98.2) | 80.7 (64.5 - 93.6) | 78.3 (60.9 - 95.7) | 78.6 (57.1 - 100.0) | 77.8 (55.6 - 100.0) | 82.9 (68.6 - 94.3) | 80.0 (64.0 - 92.0) | 90.0 (70.0 - 100.0) |
| Specificity, % (95% CI) | 85.1 (79.1 - 90.3) | 87.8 (81.7 - 93.9) | 76.9 (65.4 - 88.5) | 73.5 (55.9 - 88.2) | 81.0 (61.9 - 95.2) | 61.5 (30.8 - 84.6) | 83.6 (74.6 - 92.7) | 79.3 (65.4 - 93.1) | 92.3 (80.8 - 100.0) |
| 95% CI = 95% confidence interval<br>The TB/LTBI 42 transcript signature and TB/OD 51 transcript signature were derived using the South Africa/Malawi HIV-uninfected (HIV-) and HIV-infected (HIV+) training cohorts and then applied to the South Africa/Malawi test cohort and the independent Kenyan validation cohort. Sensitivity and specificity were calculated using weighted threshold for classification. | | | | | | | | | |

**Table 2B. Diagnostic performance of the TB/OD disease risk score in the SA/Malawi test set and the Kenyan validation cohort (culture negative subjects included) and comparison with Xpert MTB/RIF.**

| | Test set SA and Malawi | | | Kenyan Independent Validation Cohort | | | | | |
|---|---|---|---|---|---|---|---|---|---|
| | TB/OD 51 transcript signature | | | | | | Xpert MTB/RIF* | | |
| | HIV- & HIV+ combined | HIV- | HIV+ | HIV- & HIV+ combined | HIV- | HIV+ | HIV- & HIV+ combined | HIV- | HIV+ |
| Number of participants | TB=23 OD=34 | TB=14 OD=21 | TB=9 OD=13 | TB=35 OD=55 | TB=25 OD=29 | TB=10 OD=26 | TB=35 OD=55 | TB=25 OD=29 | TB=10 OD=26 |
| Area under ROC curve (95% CI) | 86.2 (77.1 - 94.0) | 88.4 (75.9 - 97.6) | 84.6 (64.0 - 96.6) | 89.0 (82.3 - 94.9) | 85.7 (75.0 - 94.4) | 93.9 (83.9 - 100.0) | 77.1 (69.9 - 85.7) | 74.0 (64.0 - 84.0) | 85.0 (70.0 - 95.1) |
| Sensitivity % (95% CI) | 78.3 (60.9 - 95.7) | 78.6 (57.1 - 100.0) | 77.8 (55.6 - 100.0) | 82.9 (68.6 - 94.3) | 80.0 (64.0 - 92.0) | 90.0 (70.0 - 100.0) | 54.3 (37.1-68.6) | 48.0 (28.0 - 64.1) | 70.0 (40.0 - 100.0) |
| Specificity % (95% CI) | 73.5 (55.9 - 88.2) | 81.0 (61.9 - 95.2) | 61.5 (30.8 - 84.6) | 83.6 (74.6 - 92.7) | 79.3 (65.4 - 93.1) | 92.3 (80.8 - 100.0) | 100.0 (100.0 - 100.0) | 100.0 (100.0- 100.0) | 100.0 (100.0- 100.0) |

* The Xpert MTB/RIF test had a positive outcome for 19 out of 35 culture-confirmed TB cases and 0 out of 55 other diseases cases.
The TB/LTBI 42 transcript signature and TB/OD 51 transcript signature were derived using the South Africa/Malawi HIV-uninfected (HIV-) and HIV-infected (HIV+) training cohorts and then applied to the South Africa/Malawi test cohort and the independent Kenyan validation cohort. Sensitivity and specificity was calculated using weighted threshold for classification. See Figure S5.

**Table 2C. Diagnostic performance of the TB/OD disease risk score and the Xpert MTB/RIF on culture-negative TB samples from the Kenyan validation cohort (culture negative included).**

| | | Area under ROC curve % (95% CI) | Sensitivity % (95% CI) | Effective Sensitivity % (95% CI) | | |
|---|---|---|---|---|---|---|
| **Highly Probable TB vs. OD** ($n_{TB}$=8 $n_{OD}$=55) | Estimated "actual" TB prevalence in group | 100% | 100% | 70% | 80% | 90% |
| | DRS 51 TB vs OD signature | 77.5 (58.2 − 94.3) | 62.5 (25.0 − 100.0) | 82.3 (41.9 − 100.0) | 74.1 (37.6 − 100.0) | 67.6 (35.1 − 100.0) |
| | Xpert MTB/RIF®* | 62.5 (50.0 − 81.3) | 25.0 (0.0 − 50.0) | 35.7 (1.1 − 65.7) | 31.3 (1.0 − 57.6) | 27.8 (1.0 − 51.3) |
| **Probable TB vs. OD** ($n_{TB}$=19 $n_{OD}$=55) | Estimated "actual" TB prevalence in group | 100% | 100% | 40% | 50% | 60% |
| | DRS 51 TB vs OD signature | 72.3 (59.6 − 84.2) | 42.1 (21.1 − 63.2) | 80.8 (36.4 − 100.0) | 67.9 (32.7 − 100.0) | 59.3 (30.2 − 90.6) |
| | Xpert MTB/RIF®* | 52.6 (50.0 − 57.9) | 5.3 (0.0 − 17.8) | 13.3 (0.0 − 36.5) | 10.6 (0.0 − 29.3) | 8.8 (0.0 − 24.5) |
| **Possible TB vs. OD** ($n_{TB}$=17 $n_{OD}$=55) | Estimated "actual" TB prevalence in group | 100% | 100% | 30% | 40% | 50% |
| | DRS 51 TB vs OD signature | 64.5 (48.4 − 77.7) | 35.3 (11.8 − 58.8) | 79.6 (7.2 − 100.0) | 63.8 (9.2 − 100.0) | 54.3 (10.2 − 91.0) |
| | Xpert MTB/RIF®* | 50.0 (50.0 − 50.0) | 0.0 (0.0 − 0.0) | 0.0 (0.0 − 0.0) | 0.0 (0.0 − 0.0) | 0.0 (0.0 − 0.0) |

* The Xpert MTB/RIF test had a positive outcome for 3 out of 44 culture-negative TB cases and 0 out of 55 other diseases cases. Specificity remains the same as in Table 2B.

**Table 3.** 42 transcript signature for distinguishing TB from latent TB infection.

| Array ID | ILMN Gene | Transcript | Direction* | Description |
|---|---|---|---|---|
| 4210411 | NDRG2 | ILMN_19545 | DOWN | NDRG family member 2 (NDRG2), transcript variant 6, mRNA. |
| 6550358 | UBA52 | ILMN_27795 | DOWN | ubiquitin A-52 residue ribosomal protein fusion product 1 (UBA52), transcript variant 2, mRNA. |
| 7150189 | PHF17 | ILMN_1535 | DOWN | PHD finger protein 17 (PHF17), transcript variant S, mRNA. |
| 6280433 | SNHG7 | ILMN_371358 | DOWN | small nucleolar RNA host gene 7 (non-protein coding) (SNHG7), transcript variant 1, non-coding RNA. |
| 1500546 | C20ORF201 | ILMN_25727 | DOWN | chromosome 20 open reading frame 201 (C20orf201), mRNA. |
| 2140541 | LOC389816 | ILMN_182870 | DOWN | cytokeratin associated protein (LOC389816), mRNA. |
| 6770603 | NOG | ILMN_7080 | DOWN | noggin (NOG), mRNA. |
| 1500575 | HS.538100 | ILMN_103699 | DOWN | xn24e12.x1 NCI_CGAP-Kid11 cDNA clone IMAGE:2694670 3, mRNA sequence |
| 5550397 | APOL6 | ILMN_38312 | UP | apolipoprotein L, 6 (APOL6), mRNA. |
| 1470706 | C8ORF55 | ILMN_25304 | DOWN | chromosome 8 open reading frame 55 (C8orf55), mRNA. |
| 6110427 | CLIP1 | ILMN_15054 | UP | CAP-GLY domain containing linker protein 1 (CLIP1), transcript variant 1, mRNA. |
| 7400341 | C11ORF2 | ILMN_10940 | DOWN | chromosome 11 open reading frame 2 (C11orf2), mRNA. |
| 3310324 | ALKBH7 | ILMN_7229 | DOWN | alkB, alkylation repair homolog 7 (E. coli) (ALKBH7), mRNA. |
| 3780047 | GBP6 | ILMN_1956 | UP | guanylate binding protein family, member 6 (GBP6), mRNA. |
| 7330575 | KLHL28 | ILMN_22112 | DOWN | kelch-like 28 (Drosophila) (KLHL28), mRNA. |
| 450632 | GNG3 | ILMN_7558 | DOWN | guanine nucleotide binding protein (G protein), gamma 3 (GNG3), mRNA. |
| 3390068 | E4F1 | ILMN_23848 | DOWN | E4F transcription factor 1 (E4F1), mRNA. |
| 2810669 | LCMT1 | ILMN_16696 | DOWN | leucine carboxyl methyltransferase 1 (LCMT1), transcript variant 1, mRNA. |
| 4260189 | TGIF1 | ILMN_162784 | DOWN | TGFB-induced factor homeobox 1 (TGIF1), transcript variant 1, mRNA. |
| 3400468 | RAP1A | ILMN_20446 | UP | RAP1A, member of RAS oncogene family (RAP1A), transcript variant 1, mRNA. |
| 2030170 | CARD16 | ILMN_21555 | UP | caspase recruitment domain family, |
|  |  |  |  | member 16 (CARD16), transcript variant 2, mRNA. |
| 4150017 | PAQR7 | ILMN_3765 | DOWN | progestin and adipoQ receptor family member VII (PAQR7), mRNA. |
| 6380187 | C21ORF57 | ILMN_21121 | DOWN | chromosome 21 open reading frame 57 (C21orf57), transcript variant 1, mRNA. |
| 2140382 | PASK | ILMN_19873 | DOWN | PAS domain containing serine/threonine kinase (PASK), mRNA. |

(continued)

| Array ID | ILMN Gene | Transcript | Direction* | Description |
|---|---|---|---|---|
| 4590026 | IMPDH2 | ILMN_3439 | DOWN | IMP (inosine monophosphate) dehydrogenase 2 (IMPDH2), mRNA. |
| 1510364 | GBP5 | ILMN_24462 | UP | guanylate binding protein 5 (GBP5), mRNA. |
| 4540239 | DEFA1 | ILMN_29692 | UP | defensin, alpha 1 (DEFA1), mRNA. |
| 4150100 | PASK | ILMN_19873 | DOWN | PAS domain containing serine/threonine kinase (PASK), mRNA. |
| 5340414 | LGTN | ILMN_4831 | DOWN | ligatin (LGTN), mRNA. |
| 5340246 | CRIP2 | ILMN_29728 | DOWN | cysteine-rich protein 2 (CRIP2), mRNA. |
| 7200274 | DGCR6 | ILMN_138781 | DOWN | DiGeorge syndrome critical region gene 6 (DGCR6), mRNA. |
| 4670487 | SIVA | ILMN_6846 | DOWN | CD27-binding (Siva) protein (SIVA), transcript variant 2, mRNA. |
| 2230538 | LRRN3 | ILMN_306943 | DOWN | leucine rich repeat neuronal 3 (LRRN3), transcript variant 1, mRNA. |
| 3440647 | DNAJC30 | ILMN_30295 | DOWN | DnaJ (Hsp40) homolog, subfamily C, member 30 (DNAJC30), mRNA. |
| 6450424 | NME3 | ILMN_23571 | DOWN | non-metastatic cells 3, protein expressed in (NME3), mRNA. |
| 4050059 | U2AF1L4 | ILMN_8757 | DOWN | U2 small nuclear RNA auxiliary factor 1-like 4 (U2AF1L4), transcript variant 2, mRNA. |
| 6480059 | ACTA2 | ILMN_6588 | UP | actin, alpha 2, smooth muscle, aorta (ACTA2), mRNA. |
| 5560075 | MFGE8 | ILMN_11368 | DOWN | milk fat globule-EGF factor 8 protein (MFGE8), mRNA. |
| 4860128 | DEFA1B | ILMN_176067 | UP | defensin, alpha 1B (DEFA1B), mRNA. |
| 4670441 | FBLN5 | ILMN_29187 | DOWN | fibulin 5 (FBLN5), mRNA. |
| 2970747 | DEFA3 | ILMN_11220 | UP | defensin, alpha 3, neutrophil-specific (DEFA3), mRNA. |
| 620403 | LOC400759 | ILMN_181219 | UP | similar to Interferon-induced guanylate-binding protein 1 (GTP-binding protein 1) (Guanine nucleotide-binding protein 1) (HuGBP-1) (LOC400759) on chromosome 1. |
| * in TB patients in relation to patients with latent TB infection. | | | | |

Table 4. 51 transcript signature for distinguishing TB from other diseases.

| Array ID | ILMN Gene | Transcript | Direction* | Description |
|---|---|---|---|---|
| 130086 | CYB561 | ILMN_8373 | UP | cytochrome b-561 (CYB561), transcript variant 1, mRNA. |
| 3780047 | GBP6 | ILMN_1956 | UP | guanylate binding protein family, member 6 (GBP6), mRNA. |
| 5340762 | HS.106234 | ILMN_74965 | UP | cDNA FLJ37173 fis, clone BRACE2028392 |
| 3390564 | CCDC52 | ILMN_23129 | UP | coiled-coil domain containing 52 (CCDC52), mRNA. |
| 2350189 | GBP3 | ILMN_3653 | UP | guanylate binding protein 3 (GBP3), mRNA. |
| 2630195 | VAMP5 | ILMN_20179 | DOWN | vesicle-associated membrane protein 5 (myobrevin) (VAMP5), mRNA. |

(continued)

| Array ID | ILMN Gene | Transcript | Direction* | Description |
|---|---|---|---|---|
| 2350121 | LOC642678 | ILMN_38908 | UP | PREDICTED: similar to myeloid/lymphoid or mixed-lineage leukemia 3 isoform 2 (LOC642678), mRNA. |
| 1070477 | ALDH1A1 | ILMN_177898 | UP | aldehyde dehydrogenase 1 family, member A1 (ALDH1A1), mRNA. |
| 3940754 | CD226 | ILMN_3877 | UP | CD226 molecule (CD226), mRNA. |
| 4290026 | C20ORF103 | ILMN_165304 | DOWN | chromosome 20 open reading frame 103 (C20orf103), mRNA. |
| 540520 | SNORD8 | ILMN_366693 | UP | small nucleolar RNA, C/D box 8 (SNORD8), small nucleolar RNA. |
| 4780044 | LOC389386 | ILMN_352098 | UP | PREDICTED: misc_RNA (LOC389386), partial miscRNA. |
| 4760747 | TPST1 | ILMN_174128 | UP | tyrosylprotein sulfotransferase 1 (TPST1), mRNA. |
| 3170246 | PDCD1LG2 | ILMN_3561 | UP | programmed cell death 1 ligand 2 (PDCD1LG2), mRNA. |
| 3940088 | ZBED2 | ILMN_4927 | DOWN | zinc finger, BED-type containing 2 (ZBED2), mRNA. |
| 160368 | SEMA6B | ILMN_21277 | DOWN | sema domain, transmembrane domain (TM), and cytoplasmic domain, (semaphorin) 6B (SEMA6B), mRNA. |
| 5890653 | CDKN1C | ILMN_20689 | DOWN | cyclin-dependent kinase inhibitor 1C (p57, Kip2) (CDKN1C), mRNA. |
| 4880370 | JUP | ILMN_3789 | DOWN | junction plakoglobin (JUP), transcript variant 1, mRNA. |
| 2600634 | C3HC4 | ILMN_6980 | DOWN | membrane-associated ring finger |
| | | | | (C3HC4) 8 (MARCH8), transcript variant 6, mRNA. |
| 6840767 | FRMD3 | ILMN_11826 | DOWN | FERM domain containing 3 (FRMD3), mRNA. |
| 460463 | SMARCD3 | ILMN_19301 | UP | SWI/SNF related, matrix associated, actin dependent regulator of chromatin, subfamily d, member 3 (SMARCD3), transcript variant 1, mRNA. |
| 5910019 | C1QB | ILMN_36274 | UP | complement component 1, q subcomponent, B chain (C1QB), mRNA. |
| 1780440 | CD79A | ILMN_37614 | UP | CD79a molecule, immunoglobulin-associated alpha (CD79A), transcript variant 1, mRNA. |
| 6510707 | FER1L3 | ILMN_18562 | UP | fer-1-like 3, myoferlin (C. elegans) (FER1L3), transcript variant 1, mRNA. |
| 2000292 | SCGB3A1 | ILMN_23096 | DOWN | secretoglobin, family 3A, member 1 (SCGB3A1), mRNA. |
| 6220739 | GRAMD1B | ILMN_308544 | DOWN | GRAM domain containing 1B (GRAMD1B), mRNA. |
| 5340767 | CEACAM1 | ILMN_21651 | DOWN | carcinoembryonic antigen-related cell adhesion molecule 1 (biliary glycoprotein) (CEACAM1), transcript variant 1, mRNA. |
| 1240554 | TNFRSF17 | ILMN_17574 | UP | tumor necrosis factor receptor superfamily, member 17 (TNFRSF17), mRNA. |
| 4570164 | LOC389386 | ILMN_165610 | UP | PREDICTED: misc_RNA (LOC389386), partial miscRNA. |

(continued)

| Array ID | ILMN Gene | Transcript | Direction* | Description |
|---|---|---|---|---|
| 840446 | CYB561 | ILMN_20474 | UP | cytochrome b-561 (CYB561), transcript variant 3, mRNA. |
| 830639 | LOC653778 | ILMN_32201 | DOWN | PREDICTED: similar to solute carrier family 25, member 37 (LOC653778), mRNA. |
| 7560114 | KLHDC8B | ILMN_6513 | UP | kelch domain containing 8B (KLHDC8B), mRNA. |
| 1400593 | SIGLEC14 | ILMN_309673 | UP | sialic acid binding Ig-like lectin 14 (SIGLEC14), mRNA. |
| 1050215 | KCNJ15 | ILMN_164363 | DOWN | potassium inwardly-rectifying channel, subfamily J, member 15 (KCNJ15), transcript variant 1, mRNA. |
| 6900291 | LOC649210 | ILMN_33006 | DOWN | PREDICTED: similar to Ig lambda |
| | | | | chain V region 4A precursor (LOC649210), mRNA. |
| 6760593 | OSBPL10 | ILMN_11112 | UP | oxysterol binding protein-like 10 (OSBPL10), mRNA. |
| 5310445 | KREMEN1 | ILMN_41914 | DOWN | kringle containing transmembrane protein 1 (KREMEN1), transcript variant 4, mRNA. |
| 620544 | HLA-DRB6 | ILMN_5312 | UP | major histocompatibility complex, class II, DR beta 6 (pseudogene) (HLA-DRB6), non-coding RNA. |
| 7320678 | HS.171481 | ILMN_80341 | UP | hx21e11.y1 Human primary human ocular pericytes. Equalized (hx) Homo sapiens cDNA clone hx21e11 5, mRNA sequence |
| 1580048 | CAST | ILMN_163108 | UP | calpastatin (CAST), transcript variant 9, mRNA. |
| 1050068 | F2RL1 | ILMN_176188 | UP | coagulation factor II (thrombin) receptor-like 1 (F2RL1), mRNA. |
| 630619 | HPSE | ILMN_165418 | DOWN | heparanase (HPSE), mRNA. |
| 2260349 | MIR1974 | ILMN_388657 | DOWN | microRNA 1974 (MIR1974), microRNA. |
| 5260484 | HLA-DRB1 | ILMN_20550 | UP | major histocompatibility complex, class II, DR beta 1 (HLA-DRB1), mRNA. |
| 1510364 | GBP5 | ILMN_24462 | UP | guanylate binding protein 5 (GBP5), mRNA. |
| 4180768 | ALAS2 | ILMN_13644 | UP | aminolevulinate, delta-, synthase 2 (ALAS2), nuclear gene encoding mitochondrial protein, transcript variant 3, mRNA. |
| 2570438 | KIFC3 | ILMN_4695 | UP | kinesin family member C3 (KIFC3), mRNA. |
| 6480364 | LOC647460 | ILMN_38026 | DOWN | PREDICTED: similar to Ig kappa chain V-1 region HK101 precursor (LOC647460), mRNA. |
| 6370315 | HLA-DRB5 | ILMN_3178 | UP | major histocompatibility complex, class II, DR beta 5 (HLA-DRB5), mRNA. |
| 4540239 | DEFA1 | ILMN_29692 | UP | defensin, alpha 1 (DEFA1), mRNA. |
| 830750 | NCF1B | ILMN_168368 | UP | neutrophil cytosolic factor 1B pseudogene (NCF1B), non-coding RNA. |

* in TB patients in relation to patients with other diseases.

**Table 5. Diagnostic performance of the disease risk score based on the TB vs. LTBI signature in the SA/Malawi test set.** Test set: nTB=23 nLTBI=11, Validation cohort: nTB=35 nLTBI=14.

| | Test set SA and Malawi | Kenya Validation Cohort (culture negative included) |
|---|---|---|
| | HIV- & HIV+ combined | |
| TB vs. LTBI 42 transcript DRS | | |
| Area under ROC curve (95% CI) | 98.4 (94.5 - 100.0) | 100.0 (100.0 - 100.0) |
| Sensitivity % (95% CI) | 95.7 (87.0 - 100.0) | 94.3 (85.7 - 100.0) |
| Specificity % (95% CI) | 90.9 (72.7 - 100.0) | 100.0 (100.0 - 100.0) |

**Table 6**. Strategy for selection of culture-negative TB. Proportion of cases arrayed was approximately equal to the proportion of expected actual TB cases assuming a 80%, 50%, 40% prevalence of TB in highly probable, probable and possible TB respectively.

| Group | Number of samples recruited | Prevalence of TB in group | Expected number of actual TB cases | Proportion of expected number of cases | Number of cases arrayed | Proportion of cases arrayed |
|---|---|---|---|---|---|---|
| Highly probable TB | 15 | 80% | 12 | 17% | 8 | 18% |
| Probable TB | 64 | 50% | 32 | 45% | 19 | 43% |
| Possible TB | 66 | 40% | 26 | 38% | 17 | 39% |
| Total | 145 | | 70 | 100% | 44 | 100% |

**Table 7a**. Comparison of culture-negative TB cases included in & excluded from the array analysis by diagnostic category

| | Highly probable TB | | | | | Probable TB | | | | | Possible TB | | | | |
|---|---|---|---|---|---|---|---|---|---|---|---|---|---|---|---|
| | Included (n=8) | | Excluded (n=7) | | p value[2] | Included (n=19) | | Excluded (n=45) | | p value[2] | Included (n=17) | | Excluded (n=49) | | p value[2] |
| **Tuberculosis exposure[1]** | | | | | | | | | | | | | | | |
| Close TB contact history | 5 | (63%) | 2 | (29%) | 0.31 | 11 | (58%) | 10 | (22%) | 0.009 | 5 | (29%) | 8 | (16%) | 0.29 |
| TST positive | 8 | (100%) | 6 | (86%) | 0.47 | 6 | (39%) | 13 | (29%) | 1 | 1 | (6%) | 1 | (2%) | 0.45 |
| Tuberculosis exposure | 8 | (100%) | 6 | (86%) | 0.47 | 13 | (68%) | 20 | (44%) | 0.10 | 5 | (29%) | 8 | (16%) | 0.29 |
| **Clinical symptoms/signs of TB[1]** | | | | | | | | | | | | | | | |
| Persistent cough >2 weeks | 5 | (63%) | 5 | (71%) | 1 | 11 | (58%) | 30 | (67%) | 0.57 | 12 | (71%) | 30 | (61%) | 0.57 |
| Persistent fever >2 weeks | 4 | (50%) | 4 | (57%) | 1 | 6 | (32%) | 27 | (60%) | 0.06 | 8 | (47%) | 33 | (67%) | 0.16 |
| Night sweats >2 weeks | 3 | (38%) | 3 | (43%) | 1 | 6 | (32%) | 7 | (16%) | 0.18 | 3 | (18%) | 10 | (20%) | 1 |
| Weight loss or failure to thrive | 7 | (88%) | 6 | (86%) | 1 | 12 | (63%) | 28 | (62%) | 1 | 12 | (71%) | 34 | (69%) | 1 |
| **CXR features of TB[1]** | | | | | | | | | | | | | | | |
| Airway compression | 1 | (13%) | 0 | (0%) | 1 | 1 | (5%) | 0 | (5%) | 0.30 | 0 | (0%) | 0 | (0%) | - |
| Lymphadenopathy | 6 | (75%) | 6 | (86%) | 1 | 7 | (37%) | 19 | (42%) | 0.78 | 1 | (6%) | 4 | (8%) | 1 |
| Airspace shadowing | 3 | (38%) | 2 | (29%) | 1 | 9 | (47%) | 18 | (40%) | 0.59 | 3 | (18%) | 15 | (31%) | 0.36 |
| Miliary/nodular shadowing | 0 | (0%) | 0 | (0%) | - | 1 | (5%) | 1 | (2%) | 0.51 | 1 | (6%) | 1 | (2%) | 0.45 |
| Pleural effusion | 0 | (0%) | 1 | (14%) | 0.47 | 2 | (11%) | 2 | (4%) | 0.58 | 0 | (0%) | 1 | (2%) | 1 |
| Cavities | 0 | (0%) | 0 | (0%) | - | 2 | (11%) | 3 | (7%) | 0.63 | 0 | (0%) | 1 | (2%) | 1 |
| Calcified Ghon focus | 0 | (0%) | 0 | (0%) | - | 0 | (0%) | 0 | (0%) | - | 0 | (0%) | 0 | (0%) | - |
| Vertebral spondylitis | 0 | (0%) | 0 | (0%) | - | 0 | (0%) | 0 | (0%) | - | 0 | (0%) | 0 | (0%) | - |

[1]See reference Zou et al 2005
[2]Fisher's exact 2-sided test

**Table 7b. Comparison of OD cases included in & excluded from the array analysis by diagnostic category**

| | Included (n=55) | | Excluded (n=935) | | p value[2] |
|---|---|---|---|---|---|
| **Tuberculosis exposure[1]** | | | | | |
| Close TB contact history | 5 | (9%) | 144 | (15%) | 0.25 |
| TST positive | 3 | (5%) | 119 | (13%) | 0.14 |
| Tuberculosis exposure | 7 | (13%) | 238 | (25%) | 0.04 |
| **Clinical symptoms/signs of TB[1]** | | | | | |
| Persistent cough >2 weeks | 22 | (40%) | 434 | (46%) | 0.41 |
| Persistent fever >2 weeks | 20 | (36%) | 380 | (41%) | 0.57 |
| Night sweats >2 weeks | 9 | (16%) | 144 | (15%) | 0.48 |
| Weight loss or failure to thrive | 43 | (78%) | 521 | (56%) | 0.001 |
| **CXR features of TB[1]** | | | | | |
| Airway compression | 0 | (0%) | 2 | (0.2%) | 1 |
| Lymphadenopathy | 4 | (7%) | 68 | (7%) | 1 |
| Airspace shadowing | 16 | (29%) | 157 | (17%) | 0.03 |
| Miliary/nodular shadowing | 0 | (0%) | 0 | (0%) | - |
| Pleural effusion | 4 | (7%) | 17 | (2%) | 0.03 |
| Cavities | 0 | (0%) | 1 | (0.1%) | 1 |
| Calcified Ghon focus | 0 | (0%) | 0 | (0%) | - |
| Vertebral spondylitis | 0 | (0%) | 0 | (0%) | - |
| [1]See reference Zou et al 2005<br>[2]Fisher's exact 2-sided test | | | | | |

**Table 8. Positive and Negative predictive value for the Kenyan validation cohort (HIV+ve & HIV-ve) (culture negative included) in different prevalence scenarios & based on the sensitivity in both culture-negative and culture-positive TB groups**

| Combined sensitivity[a] | Statistic | Prevalence | | |
|---|---|---|---|---|
| | | 10% | 30% | 50% |
| **A: 70%** | **PPV % (95% CI)** | 38.3 (23.4 - 53.3) | 70.5 (57.4 - 83.7) | 84.8 (76.6 - 943.0) |
| | **NPV % (95% CI)** | 93.6 (94.9 - 97.7) | 87.1 (82.8 - 91.5) | 74.4 (67.0 - 81.8) |
| **B: 75%** | **PPV% (95% CI)** | 41.0 (25.8 - 56.3) | 72.9 (60.4 - 85.3) | 86.2 (78.7 - 93.7) |
| | **NPV % (95% CI)** | 96.9 (95.5 - 98.2) | 89 (84.6 - 93.4) | 77.6 (69.8 - 85.4) |

(continued)

| Combined sensitivity[a] | Statistic | Prevalence | | |
|---|---|---|---|---|
| | | 10% | 30% | 50% |
| C: 82% | PPV % (95% CI) | 44.3 (28.8 - 59.8) | 75.4 (63.8 - 87.1) | 87.8 (81.0 - 94.5) |
| | NPV % (95% CI) | 97.8 (96.5 - 99.0) | 91.9 (87.6 - 96.1) | 82.9 (74.9 - 90.9) |

[a]Combined sensitivity is defined as the average sensitivity across all culture-negative and -positive TB groups. This is calculated by weighting the adjusted sensitivity calculated in each group by the relative size of each group in the Kenyan prospective cohort. This sensitivity is calculated according to three scenarios, as described in methods, and depends on an assumption as to the prevalence of 'actual' TB in each group.
A: Definite TB (100%) + HP TB (90%) +Pr TB (60%) + Pos TB (50%)
B: Definite TB (100%) + HP TB (80%) +Pr TB (50%) + Pos TB (40%)
C: Definite TB (100%) + HP TB (70%) +Pr TB (40%) + Pos TB (30%)
[b]Prevalence represents the prevalence of actual TB in the group of children to which test is given
10%: reflects the prevalence of TB in the Kenyan cohort (culture negative included)
30%: reflects the prevalence of TB from the South Africa and Malawi recruitment
50%: reflects a scenario which includes prior filtering or a combination with another test

References

WHO report 2011 Global Tuberculosis Control 2011.(http://www.who.int/tb/publications/global_report/en/)

Call to action for childhood TB. 2011. (Accessed at http://www.stoptb.org/getinvolved/ctb_cta.asp.)Perez-Velez CM, Marais BJ. Tuberculosis in children. N Engl J Med 2012;367:348-61.

Chintu C, Mudenda V, Lucas S, et al. Lung diseases at necropsy in African children dying from respiratory illnesses: a descriptive necropsy study. Lancet 2002;360:985-90.

McNally LM, Jeena PM, Gajee K, et al. Effect of age, polymicrobial disease, and maternal HIV status on treatment response and cause of severe pneumonia in South African children: a prospective descriptive study. Lancet 2007;369:1440-51.

van Well GT, Paes BF, Terwee CB, et al. Twenty years of pediatric tuberculous meningitis: a retrospective cohort study in the western cape of South Africa. Pediatrics 2009;123:e1-8.

Zar HJ, Apolles P, Argent A, et al. The etiology and outcome of pneumonia in human immunodeficiency virus-infected children admitted to intensive care in a developing country. Pediatr Crit Care Med 2001 ;2:108-12.

Cruz AT, Starke JR. Clinical manifestations of tuberculosis in children. Paediatr Respir Rev 2007;8:107-17.

Graham SM, Marais BJ, Gie RP. Clinical features and index of suspicion of tuberculosis in children. In: Schaaf HS ZA, ed. Tuberculosis a comprehensive reference: Saunders Elsevier; 2009:154-63.

Swaminathan S, Rekha B. Pediatric tuberculosis: global overview and challenges. Clin Infect Dis 2010;50 Suppl 3:S184-94.

Marais BJ, Hesseling AC, Gie RP, Schaaf HS, Enarson DA, Beyers N. The bacteriologic yield in children with intrathoracic tuberculosis. Clin Infect Dis 2006;42:e69-71.

Zar HJ, Hanslo D, Apolles P, Swingler G, Hussey G. Induced sputum versus gastric lavage for microbiological confirmation of pulmonary tuberculosis in infants and young children: a prospective study. Lancet 2005;365:130-4.

Nicol MP, Workman L, Isaacs W, et al. Accuracy of the Xpert MTB/RIF test for the diagnosis of pulmonary tuberculosis in children admitted to hospital in Cape Town, South Africa: a descriptive study. Lancet Infect Dis 2011;11:819-24.

Machingaidze S, Wiysonge CS, Gonzalez-Angulo Y, et al. The utility of an interferon gamma release assay for diagnosis of latent tuberculosis infection and disease in children: a systematic review and meta-analysis. Pediatr Infect Dis J 2011 ;30:694-700.

Mandalakas AM, Detjen AK, Hesseling AC, Benedetti A, Menzies D. Interferon-gamma release assays and childhood tuberculosis: systematic review and meta-analysis. Int J Tuberc Lung Dis 2011 ;15:1018-32.

Madhi SA, Nachman S, Violari A, et al. Primary isoniazid prophylaxis against tuberculosis in HIV-exposed children. N Engl J Med 2011;365:21-31.

Marais BJ, Gie RP, Hesseling AC, et al. A refined symptom-based approach to diagnose pulmonary tuberculosis in children. Pediatrics 2006;118:e1350-9.

Shingadia D. The diagnosis of tuberculosis. Pediatr Infect Dis J 2012;31:302-5.

Eamranond P, Jaramillo E. Tuberculosis in children: reassessing the need for improved diagnosis in global control strategies. Int J Tuberc Lung Dis 2001;5:594-603.

Kampmann B, Whittaker E, Williams A, et al. Interferon-gamma release assays do not identify more children with active tuberculosis than the tuberculin skin test. Eur Respir J 2009;33:1374-82.

Kranzer K, van Schaik N, Karmue U, et al. High prevalence of self-reported undiagnosed HIV despite high coverage of HIV testing: a cross-sectional population based sero-survey in South Africa. PLoS One 2011;6:e25244.

WHO UNICEF UNAIDS. Global HIV/AIDS response, epidemic update and health sector progress towards universal access. Geneva; 2011.

National Statistical Office. Malawi Demographic and Health Survey 2004. Zomba, Malawi: National Statistical Office; 2005 December 2005.

Nelson LJ, Wells CD. Global epidemiology of childhood tuberculosis. Int J Tuberc Lung Dis 2004;8:636-47.

Kenya National Bureau of Statistics. Kenya Demographic and Health Survey 2008-09. Nairobi; 2010.

Lesho E, Forestiero FJ, Hirata MH, et al. Transcriptional responses of host peripheral blood cells to tuberculosis infection. Tuberculosis (Edinb) 2011;91:390-9.

Lu C, Wu J, Wang H, et al. Novel biomarkers distinguishing active tuberculosis from latent infection identified by gene expression profile of peripheral blood mononuclear cells. PLoS One 2011; 6:e24290.

Metcalfe et al 2010 ("Interferon-$\gamma$ release assays for active pulmonary tuberculosis diagnosis in adults in low- and middle-income countries: systematic review and meta-analysis" The Journal of infectious diseases 204 Suppl 4).

Berry MP, Graham CM, McNab FW, et al. An interferon-inducible neutrophil-driven blood transcriptional signature in human tuberculosis. Nature 2010;466:973-7.

Denoeud F, AuryJM, Da Silva C, et al, F; Artiguenave (2008). "Annotating genomes with massive-scale RNA sequencing". Genome Biol. 9 (12): R175.

Velculescu VE, Zhang L, Vogelstein B, Kinzler KW. (1995) "Serial analysis of gene expression". Science 270 (5235): 484-7.

Irizarry RA, Hobbs B, Collin F, Beazer-Barclay YD, Antonellis KJ, Scherf U, Speed TP. Exploration, normalization, and summaries of high density oligonucleotide array probe level data. Biostatistics. 2003 Apr; 4(2):249-64.

Tusher, Virginia Goss; Tibshirani, Robert; Chu, Gilbert (2001). "Significance analysis of microarrays applied to the ionizing radiation response". Proceedings of the National Academy of Sciences of the United States of America 98 (18): 5116-5121.

Zou, H., and Hastie, T. 2005. Regularization and variable selection via the elastic net. J Roy Stat Soc Ser B 67:301-320. The relevant algorithms of the fully functioning elastic net are incorporates herein by reference.

Crampin AC, Floyd S, Mwaungulu F, et al. Comparison of two versus three smears in identifying culture-positive tuberculosis patients in a rural African setting with high HIV prevalence. Int J Tuberc Lung Dis 2001;5:994-9.

Hussain R, Kaleem A, Shahid F, et al. Cytokine profiles using whole-blood assays can discriminate between tuberculosis patients and healthy endemic controls in a BCG-vaccinated population. J Immunol Methods 2002;264:95-108.

Franken KL, Hiemstra HS, van Meijgaarden KE, et al. Purification of his-tagged proteins by immobilized chelate affinity chromatography: the benefits from the use of organic solvent. Protein Expr Purif 2000;18:95-9.

Benjamini Y, Hochberg Y. Controlling the False Discovery Rate - a Practical and Powerful Approach to Multiple Testing. J Roy Stat Soc B Met 1995;57:289-300.

Joosten SA, Goeman JJ, Sutherland JS, et al. Identification of biomarkers for tuberculosis disease using a novel dual-color RT-MLPA assay. Genes Immun 2012;13:71-82.

Eldering E, Spek CA, Aberson HL, et al. Expression profiling via novel multiplex assay allows rapid assessment of gene regulation in defined signalling pathways. Nucleic Acids Res 2003;31:e153.

Maertzdorf J, Ota M, Repsilber D, et al. Functional correlations of pathogenesis-driven gene expression signatures in tuberculosis. PLoS One 2011a;6:e26938.

Maertzdorf J, Repsilber D, Parida SK, et al. Human gene expression profiles of susceptibility and resistance in tuberculosis. Genes Immun 2011b;12:15-22.

Jacobsen M, Repsilber D, Gutschmidt A, et al. Candidate biomarkers for discrimination between infection and disease caused by Mycobacterium tuberculosis. J Mol Med (Berl) 2007;85:613-21.

Cox JA, Lukande RL, Lucas S, Nelson AM, Van Marck E, Colebunders R. Autopsy causes of death in HIV-positive individuals in sub-Saharan Africa and correlation with clinical diagnoses. AIDS Rev 2010;12:183-94.

Ansari NA, Kombe AH, Kenyon TA, et al. Pathology and causes of death in a group of 128 predominantly HIV-positive patients in Botswana, 1997-1998. Int J Tuberc Lung Dis 2002;6:55-63.

Maertzdorf J, Weiner J, 3rd, Mollenkopf HJ, et al. Common patterns and disease-related signatures in tuberculosis and sarcoidosis. Proc Natl Acad Sci U S A 2012;109:7853-8.

Graham SM, Ahmed T, Amanullah F, et al. Evaluation of tuberculosis diagnostics in children: 1. Proposed clinical case definitions for classification of intrathoracic tuberculosis disease. Consensus from an expert panel. J Infect Dis 2012;205 Suppl 2:S199-208.

Hesseling AC, Schaaf HS, Gie RP, Starke JR, Beyers N. A critical review of diagnostic approaches used in the diagnosis of childhood tuberculosis. Int J Tuberc Lung Dis 2002;6:1038-45.

Hatherill M, Hanslo M, Hawkridge T, et al. Structured approaches for the screening and diagnosis of childhood tuberculosis in a high prevalence region of South Africa. Bull World Health Organ 2010;88:312-20.

Pearce EC, Woodward JF, Nyandiko WM, Vreeman RC, Ayaya SO. A systematic review of clinical diagnostic systems used in the diagnosis of tuberculosis in children. AIDS Res Treat 2012;2012:401896.

Cuevas L, Petrucci R, Swaminathan S. Tuberculosis diagnostics for children in high-burden countries: what is available and what is needed. Paediatr Child Health 2012;32:30-7.

Drobac PC, Shin SS, Huamani P, et al. Risk factors for in-hospital mortality among children with tuberculosis: the 25-year experience in Peru. Pediatrics 2012;130:e373-9.

Marais BJ, Gie RP, Schaaf HS, et al. The natural history of childhood intra-thoracic tuberculosis: a critical review of literature from the pre-chemotherapy era. Int J Tuberc Lung Dis 2004;8:392-402.

**Claims**

1. A method for detecting active TB in a sample derived from a child in the presence of a complicating factor, comprising the step of measuring RNA levels in the sample of at least 95% of the genes in a signature selected from the group consisting of:

   a) a 42 gene signature shown in Table 3 for discriminating active TB from latent TB infection,
   b) a 51 gene signature shown in Table 4 for discriminating active TB from other diseases,
   c) a combination of signatures a) and b) and detecting the modulation in gene expression data, generated from said RNA levels.

2. A method according to Claim 1 wherein 100% of the genes of a given signature are detected.

3. A method according to any one of Claims 1 to 2 wherein the gene expression data is generated from a microarray.

4. A method according to any one of Claims 1 to 3 wherein the detection method employs fluorescence or colorimetric analysis.

5. A method according to any one of claims 1 to 4 wherein the complicating factor is latent TB.

6. A method according to any one of Claims 1 to 5 wherein the complicating factors is the presence of a co-morbidity, for example wherein the co-morbidity is selected from malignancy, HIV, malaria, pneumonia, lower respiratory tract infection, pneumocystis, Jirovecii Pneumonia, pelvic inflammatory disease, urinary tract infection, bacterial or vial meningitis, hepatobiliary disease, cryptococcal meningitis, non-TB pleural effusion, empyema, gastroenteritis, peritonitis, gastric ulcer and gastritis.

7. A method according to Claim 6 wherein the co-morbidity is HIV or malaria.

8. A method according to claim 6, wherein the malignancy is a neoplasia, such as bronchial carcinoma, lymphoma, cervical carcinoma, ovarian carcinoma, mesothelioma, gastric carcinoma, metastatic carcinoma, benign salivary tumour, dermatological tumour or Kaposi's sarcoma.

9. A method according to any one of Claims 1 to 8 wherein 11 genes in the 42 gene signature are up-regulated wherein the 11 genes are APOL6, CLIPI, GBP6, AP1A, CARD16, GBP5, DEFA1, ACTA2, DEFA1B, DEFA3 and LOC400759.

10. A method according to claim 9 wherein the remaining genes in the signature are down-regulated wherein remaining genes are NDRG2, UBA52, PHF17, SNHG7, C20ORF201, LOC389816, NOG, HS.538100, C80RF55, C110RF2, ALKBH7, KLHL28, GNG3, E4F1, LCMT1, TGIF1, PAQR7, C210RF57, PASK, IMPDH2, PASK, LGTN, CRIP2, DGCR6, SIVA, LRRN3, DNAJC30, NME3, U2AF1L4, MFGE8 and FBLN5.

11. A method according to any one of Claims 1 to 10 wherein 33 genes in the 51 gene signature are up-regulated wherein the 33 genes are CYB561, GBP6, HS.106234, CCDC52, GBP3, LOC642678, ALDH1A1, CD226, SNORD8, LOC389386, TPST1, PDCD1LG2, SMARCD3, C1QB, CD79A, FER1L3, TNFRSF17, LOC389386, CYB561,KLHDC8B, SIGLEC14, OSBPL10, HLA-DRB6, HS.171481, CAST, F2RL1, HLA-DRB1, GBP5, ALAS2, KIFC3, HLA-DRB5, DEFA1 and NCF1B.

12. A method according to Claim 11, wherein the remaining genes are in the signature are down- regulated wherein the remaining genes are VAMP5, C20ORF103, ZBED2, SEMA6B, CDKN1C, JUP, C3HC4, FRMD3, SCGB3A1, GRAMD1B, CEACAM1, LOC653778, KCNJ15, LOC649210, KREMEN1, HPSE, MIR1974 and LOC647460.

13. A method according to any one of Claims 1 to 12 further comprising the steps of:

a. optionally normalising and/or scaling numeric values of the modulation b. taking the normalised and/or scaled numeric values or the raw numeric values, each of which comprise both positive and/or negative numeric values and designating all said numeric values to be negative or alternatively all positive,

c. optionally refining the discriminatory power of one or more up-regulated genes and down-regulated genes by statistically weighting some of the numeric values associated therewith, and d. summating the positive or negative numeric values obtained from step b) or step c) to provide a composite expression score,

wherein the composite expression score obtained from step d) is compared to a control and the comparison allows the sample to be designated as positive or negative for the relevant infection.

14. A gene chip consisting of nucleic acid probes for all the genes of one or more of the gene signatures selected from the group consisting of:

    a) a 42 gene signature shown in Table 3,
    b) a 51 gene signature shown in Table 4, and
    c) a combination of signatures a) and b).

**Patentansprüche**

1. Verfahren zum Nachweisen von aktiver TB in einer Probe, die bei Vorliegen eines verkomplizierenden Faktors von einem Kind gewonnen wird, umfassend den Schritt des Messens von RNA-Spiegeln von wenigstens 95 % der Gene in der Probe in einer Signatur, die ausgewählt ist aus der Gruppe bestehend aus:

    a) einer 42-Gen-Signatur, dargestellt in Tabelle 3, zum Unterscheiden von aktiver TB von latenter TB-Infektion,
    b) einer 51-Gen-Signatur, dargestellt in Tabelle 4, zum Unterscheiden von aktiver TB von anderen Erkrankungen,
    c) einer Kombination von Signatur a) und b) und Nachweisen der Modulation in Genexpressionsdaten, die aus den RNA-Spiegeln generiert werden.

2. Verfahren nach Anspruch 1, wobei 100 % der Gene einer gegebenen Signatur nachgewiesen werden.

3. Verfahren nach einem der Ansprüche 1 bis 2, wobei die Genexpressionsdaten aus einem Mikroarray generiert werden.

4. Verfahren nach einem der Ansprüche 1 bis 3, wobei das Nachweisverfahren Fluoreszenz- oder kolorimetrische Analyse nutzt.

5. Verfahren nach einem der Ansprüche 1 bis 4, wobei der verkomplizierende Faktor latente TB ist.

6. Verfahren nach einem der Ansprüche 1 bis 5, wobei die verkomplizierenden Faktoren das Vorliegen einer Begleiterkrankung ist, zum Beispiel wobei die Begleiterkrankung ausgewählt ist aus Malignität, HIV, Malaria, Pneumonie, Infekt der unteren Atemwege, Pneumocystis, Jirovecii-Pneumonie, entzündlicher Beckenerkrankung, Harnwegsinfekt, bakterieller oder viraler Meningitis, hepatobiliärer Erkrankung, Kryptokokkenmeningitis, Nicht-TB-Pleuraerguss, Empyem, Gastroenteritis, Peritonitis, Magengeschwür und Gastritis.

7. Verfahren nach Anspruch 6, wobei die Begleiterkrankung HIV oder Malaria ist.

8. Verfahren nach Anspruch 6, wobei die Malignität eine Neoplasie ist, wie etwa Bronchialkarzinom, Lymphom, Zervixkarzinom, Ovarialkarzinom, Mesotheliom, Magenkarzinom, metastatisches Karzinom, benigner Speicheldrüsentumor, Hauttumor oder Kaposi-Sarkom.

9. Verfahren nach einem der Ansprüche 1 bis 8, wobei 11 Gene in der 42-Gen-Signatur hochreguliert sind, wobei die 11 Gene APOL6, CLIPI, GBP6, AP1A, CARD16, GBP5, DEFA1, ACTA2, DEFA1B, DEFA3 und LOC400759 sind.

10. Verfahren nach Anspruch 9, wobei die restlichen Gene in der Signatur herunterreguliert sind, wobei die restlichen Gene NDRG2, UBA52, PHF17, SNHG7, C20ORF201, LOC389816, NOG, HS.538100, C80RF55, C110RF2, ALKBH7, KLHL28, GNG3, E4F1, LCMT1, TGIF1, PAQR7, C210RF57, PASK, IMPDH2, PASK, LGTN, CRIP2, DGCR6, SIVA, LRRN3, DNAJC30, NME3, U2AF1L4, MFGE8 und FBLN5 sind.

**11.** Verfahren nach einem der Ansprüche 1 bis 10, wobei 33 Gene in der 51-Gen-Signatur hochreguliert sind, wobei die 33 Gene CYB561, GBP6, HS.106234, CCDC52, GBP3, LOC642678, ALDH1A1, CD226, SNORD8, LOC389386, TPST1, PDCD1LG2, SMARCD3, C1QB, CD79A, FER1L3, TNFRSF17, LOC389386, CYB561, KLHDC8B, SIGLEC14, OSBPL10, HLA-DRB6, HS.171481, CAST, F2RL1, HLA-DRB1, GBP5, ALAS2, KIFC3, HLA-DRB5, DEFA1 und NCF1B sind.

**12.** Verfahren nach Anspruch 11, wobei die restlichen Gene in der Signatur herunterreguliert sind, wobei die restlichen Gene VAMP5, C20ORF103, ZBED2, SEMA6B, CDKN1C, JUP, C3CH4, FRMD3, SCGB3A1, GRAMD1B, CEACAM1, LOC653778, KCNJ15, LOC649210, KREMEN1, HPSE, MIR1974 und LOC647460 sind.

**13.** Verfahren nach einem der Ansprüche 1 bis 12, weiter umfassend die Schritte des:

a. gegebenenfalls Normalisierens und/oder Skalierens von Zahlenwerten der Modulation,
b. Nehmens der normalisierten und/oder skalierten Zahlenwerte oder der rohen Zahlenwerte, von denen jeder sowohl positive und/oder negative Zahlenwerte umfasst, und Benennen aller der Zahlenwerte dazu, negativ oder alternativ alle positiv zu sein,
c. gegebenenfalls Verfeinerns der Unterscheidungsleistung von einem oder mehreren hochregulierten Genen und herunterregulierten Genen durch statistisches Gewichten einiger der damit verknüpften Zahlenwerte, und
d. Summierens der erhaltenen positiven oder negativen Zahlenwerte aus Schritt b) oder Schritt c), um einen Gesamt-Expressionsscore bereitzustellen,

wobei der erhaltene Gesamt-Expressionsscore aus Schritt d) mit einer Kontrolle verglichen wird und der Vergleich der Probe ermöglicht, als positiv oder negativ auf die betreffende Infektion benannt zu werden.

**14.** Genchip, bestehend aus Nukleinsäuresonden für alle die Gene aus einer oder mehreren der Gen-Signaturen, ausgewählt aus der Gruppe bestehend aus:

a) einer 42-Gen-Signatur, dargestellt in Tabelle 3,
b) einer 51-Gen-Signatur, dargestellt in Tabelle 4, und
c) einer Kombination von Signatur a) und b).

**Revendications**

**1.** Procédé de détection de tuberculose active dans un échantillon dérivé d'un enfant en présence d'un facteur de complication, comprenant l'étape de mesure de taux d'ARN dans l'échantillon d'au moins 95 % des gènes dans une signature choisie dans le groupe constitué de :

a) une signature de 42 gènes montrée dans le Tableau 3 pour distinguer une tuberculose active d'une infection tuberculeuse latente,
b) une signature de 51 gènes montrée dans le Tableau 4 pour distinguer une tuberculose active d'autres maladies,
c) une combinaison de signature a) et b) et la détection de la modulation des données d'expression de gène, générée à partir desdits taux d'ARN.

**2.** Procédé selon la revendication 1, dans lequel 100 % des gènes d'une signature donnée sont détectés.

**3.** Procédé selon l'une quelconque des revendications 1 à 2, dans lequel les données d'expression de gène sont générées à partir d'une micromatrice.

**4.** Procédé selon l'une quelconque des revendications 1 à 3, dans lequel le procédé de détection fait appel à une analyse par fluorescence ou colorimétrique.

**5.** Procédé selon l'une quelconque des revendications 1 à 4, dans lequel le facteur de complication est une tuberculose latente.

**6.** Procédé selon l'une quelconque des revendications 1 à 5, dans lequel le facteur de complication est la présence d'une co-morbidité, par exemple, dans lequel la co-morbidité est choisie parmi une malignité, le VIH, la malaria, une pneumonie, une infection du système respiratoire inférieur, une pneumocystose, Jirovecii Pneumonia, une maladie inflammatoire pelvienne, une infection du système urinaire, une méningite bactérienne ou virale, une maladie hépatobiliaire, une méningite à cryptocoque, un épanchement pleural non tuberculeux, un empyème, une gastro-entérite, une péritonite, un ulcère gastrique et une gastrite.

7. Procédé selon la revendication 6, dans lequel la co-morbidité est le VIH ou la malaria.

8. Procédé selon la revendication 6, dans lequel la malignité est une néoplasie, telle qu'un carcinome bronchique, un lymphome, un carcinome du col de l'utérus, un carcinome ovarien, un mésothéliome, un carcinome gastrique, un carcinome métastatique, une tumeur salivaire bénigne, une tumeur dermatologique ou un sarcome de Kaposi.

9. Procédé selon l'une quelconque des revendications 1 à 8, dans lequel 11 gènes dans la signature de 42 gènes sont régulés à la hausse, dans lequel les 11 gènes sont APOL6, CLIPI, GBP6, AP1A, CARD16, GBP5, DEFA1, ACTA2, DEFA1B, DEFA3 et LOC400759.

10. Procédé selon la revendication 9, dans lequel les gènes restants dans la signature sont régulés à la baisse, dans lequel les gènes restants sont NDRG2, UBA52, PHF17, SNHG7, C20ORF201, LOC389816, NOG, HS.538100, C80RF55, C110RF2, ALKBH7, KLHL28, GNG3, E4F1, LCMT1, TGIF1, PAQR7, C210RF57, PASK, IMPDH2, PASK, LGTN, CRIP2, DGCR6, SIVA, LRRN3, DNAJC30, NME3, U2AF1L4, MFGE8 et FBLN5.

11. Procédé selon l'une quelconque des revendications 1 à 10, dans lequel 33 gènes dans la signature de 51 gènes sont régulés à la hausse, dans lequel les 33 gènes sont CYB561, GBP6, HS.106234, CCDC52, GBP3, LOC642678, ALDH1A1, CD226, SNORD8, LOC389386, TPST1, PDCD1LG2, SMARCD3, C1QB, CD79A, FER1L3, TNFRSF17, LOC389386, CYB561, KLHDC8B, SIGLEC14, OSBPL10, HLA-DRB6, HS.171481, CAST, F2RL1, HLA-DRB1, GBP5, ALAS2, KIFC3, HLA-DRB5, DEFA1 et NCF1B.

12. Procédé selon la revendication 11, dans lequel les gènes restants dans la signature sont régulés à la baisse, dans lequel les gènes restants sont VAMP5, C20ORF103, ZBED2, SEMA6B, CDKN1C, JUP, C3HC4, FRMD3, SCGB3A1, GRAMD1B, CEACAM1, LOC653778, KCNJ15, LOC649210, KREMEN1, HPSE, MIR1974 et LOC647460.

13. Procédé selon l'une quelconque des revendications 1 à 12, comprenant en outre les étapes consistant à :

    a. éventuellement normaliser et/ou mettre à l'échelle les valeurs numériques de la modulation
    b. prendre les valeurs numériques normalisées et/ou mises à l'échelle ou les valeurs numériques brutes, chacune de celles-ci comprenant à la fois des valeurs numériques positives et/ou négatives et désigner toutes lesdites valeurs numériques pour qu'elles soient négatives ou, selon une autre possibilité, toutes positives,
    c. éventuellement affiner le pouvoir de distinction d'un ou plusieurs gènes régulés à la hausse et gènes régulés à la baisse en pondérant statistiquement certaines des valeurs numériques associées à ceux-ci, et
    d. sommer les valeurs numériques positives ou négatives obtenues par l'étape b) ou l'étape c) pour fournir une cote d'expression composite,

    dans lequel la cote d'expression composite obtenue par l'étape d) est comparée à un témoin et la comparaison permet à l'échantillon d'être désigné comme étant positif ou négatif pour l'infection concernée.

14. Puce génique constituée de sondes d'acide nucléique pour tous les gènes d'une ou plusieurs parmi les signatures de gène choisies dans le groupe constitué de :

    a) une signature de 42 gènes montrée dans le Tableau 3,
    b) une signature de 51 gènes montrée dans le Tableau 4, et
    c) une combinaison de signatures a) et b).

**Figure 1A.**

* *IGRA repeated on suspected TB cases with negative IGRA at recruitment*

**Figure 1B.**

Figure 2A.

Figure 2B.

**Figure 3.**

A.

TB/LTBI
42 transcript signature

B.

TB/OD
51 transcript signature

Figure 4.

**Figure 5.**

**Figure 6.**

A.

**Figure 7.**

Figure 8A.

Figure 8B.

**Figure 8C.**

EP 2 914 740 B1

EP 2 914 740 B1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *WHO report 2011 Global Tuberculosis Control,* 2011, http://www.who.int/tb/publications/global_report/en
- **PEREZ-VELEZ CM ; MARAIS BJ.** Tuberculosis in children. *N Engl J Med,* 2012, vol. 367, 348-61
- **CHINTU C ; MUDENDA V ; LUCAS S et al.** Lung diseases at necropsy in African children dying from respiratory illnesses: a descriptive necropsy study. *Lancet,* 2002, vol. 360, 985-90
- **MCNALLY LM ; JEENA PM ; GAJEE K et al.** Effect of age, polymicrobial disease, and maternal HIV status on treatment response and cause of severe pneumonia in South African children: a prospective descriptive study. *Lancet,* 2007, vol. 369, 1440-51
- **VAN WELL GT ; PAES BF ; TERWEE CB et al.** Twenty years of pediatric tuberculous meningitis: a retrospective cohort study in the western cape of South Africa. *Pediatrics,* 2009, vol. 123, e1-8
- **ZAR HJ ; APOLLES P ; ARGENT A et al.** The etiology and outcome of pneumonia in human immunodeficiency virus-infected children admitted to intensive care in a developing country. *Pediatr Crit Care Med,* 2001, vol. 2, 108-12
- **CRUZ AT ; STARKE JR.** Clinical manifestations of tuberculosis in children. *Paediatr Respir Rev,* 2007, vol. 8, 107-17
- Clinical features and index of suspicion of tuberculosis in children. **GRAHAM SM ; MARAIS BJ ; GIE RP.** Tuberculosis a comprehensive reference. Saunders Elsevier, 2009, 154-63
- **SWAMINATHAN S ; REKHA B.** Pediatric tuberculosis: global overview and challenges. *Clin Infect Dis,* 2010, vol. 50 (3), 184-94
- **MARAIS BJ ; HESSELING AC ; GIE RP ; SCHAAF HS ; ENARSON DA ; BEYERS N.** The bacteriologic yield in children with intrathoracic tuberculosis. *Clin Infect Dis,* 2006, vol. 42, e69-71
- **ZAR HJ ; HANSLO D ; APOLLES P ; SWINGLER G ; HUSSEY G.** Induced sputum versus gastric lavage for microbiological confirmation of pulmonary tuberculosis in infants and young children: a prospective study. *Lancet,* 2005, vol. 365, 130-4
- **NICOL MP ; WORKMAN L ; ISAACS W et al.** Accuracy of the Xpert MTB/RIF test for the diagnosis of pulmonary tuberculosis in children admitted to hospital in Cape Town, South Africa: a descriptive study. *Lancet Infect Dis,* 2011, vol. 11, 819-24

- **MACHINGAIDZE S ; WIYSONGE CS ; GONZALEZ-ANGULO Y et al.** The utility of an interferon gamma release assay for diagnosis of latent tuberculosis infection and disease in children: a systematic review and meta-analysis. *Pediatr Infect Dis J,* 2011, vol. 30, 694-700
- **MANDALAKAS AM ; DETJEN AK ; HESSELING AC ; BENEDETTI A ; MENZIES D.** Interferon-gamma release assays and childhood tuberculosis: systematic review and meta-analysis. *Int J Tuberc Lung Dis,* 2011, vol. 15, 1018-32
- **MADHI SA ; NACHMAN S ; VIOLARI A et al.** Primary isoniazid prophylaxis against tuberculosis in HIV-exposed children. *N Engl J Med,* 2011, vol. 365, 21-31
- **MARAIS BJ ; GIE RP ; HESSELING AC et al.** A refined symptom-based approach to diagnose pulmonary tuberculosis in children. *Pediatrics,* 2006, vol. 118, e1350-9
- **SHINGADIA D.** The diagnosis of tuberculosis. *Pediatr Infect Dis J,* 2012, vol. 31, 302-5
- **EAMRANOND P ; JARAMILLO E.** Tuberculosis in children: reassessing the need for improved diagnosis in global control strategies. *Int J Tuberc Lung Dis,* 2001, vol. 5, 594-603
- **KAMPMANN B ; WHITTAKER E ; WILLIAMS A et al.** Interferon-gamma release assays do not identify more children with active tuberculosis than the tuberculin skin test. *Eur Respir J,* 2009, vol. 33, 1374-82
- **KRANZER K ; VAN SCHAIK N ; KARMUE U et al.** High prevalence of self-reported undiagnosed HIV despite high coverage of HIV testing: a cross-sectional population based sero-survey in South Africa. *PLoS One,* 2011, vol. 6, e25244
- Global HIV/AIDS response, epidemic update and health sector progress towards universal access. *WHO UNICEF UNAIDS,* 2011
- Malawi Demographic and Health Survey 2004. *National Statistical Office,* December 2005
- **NELSON LJ ; WELLS CD.** Global epidemiology of childhood tuberculosis. *Int J Tuberc Lung Dis,* 2004, vol. 8, 636-47
- Kenya Demographic and Health Survey 2008-09. *Kenya National Bureau of Statistics,* 2010
- **LESHO E ; FORESTIERO FJ ; HIRATA MH et al.** Transcriptional responses of host peripheral blood cells to tuberculosis infection. *Tuberculosis (Edinb),* 2011, vol. 91, 390-9

54

- **LU C ; WU J ; WANG H et al.** Novel biomarkers distinguishing active tuberculosis from latent infection identified by gene expression profile of peripheral blood mononuclear cells. *PLoS One,* 2011, vol. 6, e24290
- **METCALFE et al.** Interferon-γ release assays for active pulmonary tuberculosis diagnosis in adults in low- and middle-income countries: systematic review and meta-analysis. *The Journal of infectious diseases,* 2010, vol. 204 (4
- **BERRY MP ; GRAHAM CM ; MCNAB FW et al.** An interferon-inducible neutrophil-driven blood transcriptional signature in human tuberculosis. *Nature,* 2010, vol. 466, 973-7
- **DENOEUD F ; AURYJM ; DA SILVA C et al.** Annotating genomes with massive-scale RNA sequencing. *Genome Biol,* 2008, vol. 9 (12), R175
- **VELCULESCU VE ; ZHANG L ; VOGELSTEIN B ; KINZLER KW.** Serial analysis of gene expression. *Science,* 1995, vol. 270 (5235), 484-7
- **IRIZARRY RA ; HOBBS B ; COLLIN F ; BEAZER-BARCLAY YD ; ANTONELLIS KJ ; SCHERF U ; SPEED TP.** Exploration, normalization, and summaries of high density oligonucleotide array probe level data. *Biostatistics,* April 2003, vol. 4 (2), 249-64
- **TUSHER, VIRGINIA GOSS ; TIBSHIRANI, ROBERT ; CHU, GILBERT.** Significance analysis of microarrays applied to the ionizing radiation response. *Proceedings of the National Academy of Sciences of the United States of America,* 2001, vol. 98 (18), 5116-5121
- **ZOU, H. ; HASTIE, T.** Regularization and variable selection via the elastic net. *J Roy Stat Soc Ser B,* 2005, vol. 67, 301-320
- **CRAMPIN AC ; FLOYD S ; MWAUNGULU F et al.** Comparison of two versus three smears in identifying culture-positive tuberculosis patients in a rural African setting with high HIV prevalence. *Int J Tuberc Lung Dis,* 2001, vol. 5, 994-9
- **HUSSAIN R ; KALEEM A ; SHAHID F et al.** Cytokine profiles using whole-blood assays can discriminate between tuberculosis patients and healthy endemic controls in a BCG-vaccinated population. *J Immunol Methods,* 2002, vol. 264, 95-108
- **FRANKEN KL ; HIEMSTRA HS ; VAN MEIJGAARDEN KE et al.** Purification of his-tagged proteins by immobilized chelate affinity chromatography: the benefits from the use of organic solvent. *Protein Expr Purif,* 2000, vol. 18, 95-9
- **BENJAMINI Y ; HOCHBERG Y.** Controlling the False Discovery Rate - a Practical and Powerful Approach to Multiple Testing. *J Roy Stat Soc B Met,* 1995, vol. 57, 289-300
- **JOOSTEN SA ; GOEMAN JJ ; SUTHERLAND JS et al.** Identification of biomarkers for tuberculosis disease using a novel dual-color RT-MLPA assay. *Genes Immun,* 2012, vol. 13, 71-82
- **ELDERING E ; SPEK CA ; ABERSON HL et al.** Expression profiling via novel multiplex assay allows rapid assessment of gene regulation in defined signalling pathways. *Nucleic Acids Res,* 2003, vol. 31, e153
- **MAERTZDORF J ; OTA M ; REPSILBER D et al.** Functional correlations of pathogenesis-driven gene expression signatures in tuberculosis. *PLoS One,* 2011, vol. 6, e26938
- **MAERTZDORF J ; REPSILBER D ; PARIDA SK et al.** Human gene expression profiles of susceptibility and resistance in tuberculosis. *Genes Immun,* 2011, vol. 12, 15-22
- **JACOBSEN M ; REPSILBER D ; GUTSCHMIDT A et al.** Candidate biomarkers for discrimination between infection and disease caused by Mycobacterium tuberculosis. *J Mol Med (Berl),* 2007, vol. 85, 613-21
- **COX JA ; LUKANDE RL ; LUCAS S ; NELSON AM ; VAN MARCK E ; COLEBUNDERS R.** Autopsy causes of death in HIV-positive individuals in sub-Saharan Africa and correlation with clinical diagnoses. *AIDS Rev,* 2010, vol. 12, 183-94
- **ANSARI NA ; KOMBE AH ; KENYON TA et al.** Pathology and causes of death in a group of 128 predominantly HIV-positive patients in Botswana, 1997-1998. *Int J Tuberc Lung Dis,* 2002, vol. 6, 55-63
- **MAERTZDORF J ; WEINER J ; 3RD ; MOLLENKOPF HJ et al.** Common patterns and disease-related signatures in tuberculosis and sarcoidosis. *Proc Natl Acad Sci U S A,* 2012, vol. 109, 7853-8
- **GRAHAM SM ; AHMED T ; AMANULLAH F et al.** Evaluation of tuberculosis diagnostics in children: 1. Proposed clinical case definitions for classification of intrathoracic tuberculosis disease. Consensus from an expert panel. *J Infect Dis,* 2012, vol. 205 (2), S199-208
- **HESSELING AC ; SCHAAF HS ; GIE RP ; STARKE JR ; BEYERS N.** A critical review of diagnostic approaches used in the diagnosis of childhood tuberculosis. *Int J Tuberc Lung Dis,* 2002, vol. 6, 1038-45
- **HATHERILL M ; HANSLO M ; HAWKRIDGE T et al.** Structured approaches for the screening and diagnosis of childhood tuberculosis in a high prevalence region of South Africa. *Bull World Health Organ,* 2010, vol. 88, 312-20
- **PEARCE EC ; WOODWARD JF ; NYANDIKO WM ; VREEMAN RC ; AYAYA SO.** A systematic review of clinical diagnostic systems used in the diagnosis of tuberculosis in children. *AIDS Res Treat 2012,* 2012, 401896
- **CUEVAS L ; PETRUCCI R ; SWAMINATHAN S.** Tuberculosis diagnostics for children in high-burden countries: what is available and what is needed. *Paediatr Child Health,* 2012, vol. 32, 30-7

- **DROBAC PC ; SHIN SS ; HUAMANI P et al.** Risk factors for in-hospital mortality among children with tuberculosis: the 25-year experience in Peru. *Pediatrics,* 2012, vol. 130, e373-9

- **MARAIS BJ ; GIE RP ; SCHAAF HS et al.** The natural history of childhood intra-thoracic tuberculosis: a critical review of literature from the pre-chemotherapy era. *Int J Tuberc Lung Dis,* 2004, vol. 8, 392-402